# EUROPEAN PATENT SPECIFICATION

(11) **EP 3 336 179 B1**
(45) Date of publication and mention of the grant of the patent: **05.02.2020**
(21) Application number: 17206585.6
(22) Date of filing: 12.12.2017
(51) Int. Cl.: C12N 9/06

(54) **RECOMBINANT PROTEIN AND PREPARATION METHOD AND APPLICATION THEREOF**
REKOMBINANTES PROTEIN UND HERSTELLUNGSVERFAHREN UND ANWENDUNG DAVON
PROTÉINE RECOMBINANTE ET SON PROCÉDÉ DE PRÉPARATION ET D'APPLICATION

(30) Priority: 13.12.2016 US 201662433733 P
(43) Date of publication of application: 20.06.2018
(73) Proprietor: Industrial Technology Research Institute, Chutung, Hsinchu 31040 (TW)
(72) Inventor: HUANG, Yi-Chau, Hsinchu County 310 (TW); LEE, Huai-Lo, Hsinchu County 310 (TW); TSAI, Yu-Yin, Hsinchu City 300 (TW); LIN, Chih-Lung, Taichung City 401 (TW)
(74) Representative: Becker Kurig Straus

(56) References cited:
- EP-A1- 2 287 295
- EP-A1- 2 354 224
- MIHO KAMEYA ET AL: "Advancing the Development of Glycated Protein Biosensing Technology : Next-Generation Sensing Molecules", JOURNAL OF DIABETES SCIENCE AND TECHNOLOGY, vol. 9, no. 2, 17 March 2015 (2015-03-17), pages 183-191, XP055460586, US ISSN: 1932-2968, DOI: 10.1177/1932296814565784
- HIROKAWA K ET AL: "Molecular cloning and expression of novel fructosyl peptide oxidases and their application for the measurement of glycated protein", BIOCHEMICAL AND BIOPHYSICAL RESEARCH COMMUNICAT, ELSEVIER, AMSTERDAM, NL, vol. 311, no. 1, 7 November 2003 (2003-11-07), pages 104-111, XP004465110, ISSN: 0006-291X, DOI: 10.1016/J.BBRC.2003.09.169

## Description

This application claims the priority benefits of U.S. provisional application serial no. 62/433,733, filed on December 13, 2016.

### SEQUENCE LISTING

A sequence listing submitted as a text file via EFS-Web containing the sequence listing is named "66204EP_SEQUENCE_LISTING.txt"; its date of creation is December 7, 2017; and its size is 37 kilobytes.

### TECHNICAL FIELD

The disclosure relates to a recombinant protein and a preparation method and application thereof, and more particularly, to a recombinant protein suitable for detecting glycated hemoglobin.

### BACKGROUND

For diabetics, the evaluation of the treatment or control effect of blood glucose includes using hemoglobin Alc (HbAlc) as the best detection target, and an enzyme method is used as the routine clinical detection method. The detection principle of the enzyme method is based on the glycolysis of fructosyl amino acid oxidase (FAOD), in which FAOD is used as the main reaction enzyme, and fructosyl valine (F-V) and fructosyl valyl histidine (F-VH) are used as the reaction substrates to perform the detection of glycated hemoglobin.

Therefore, during glycated hemoglobin detection using an enzyme method, substrate specificity of the fructosyl amino acid oxidase mainly acting as the main reaction enzyme is an essential factor. However, during the hydrolysis of glycated hemoglobin by protease, in addition to the production of F-V and F-VH, a large quantity of fructosyl lysine (F-K) is also relatively produced, and the current FAOD on the market still has a certain affinity for F-K, such that the detection of glycated hemoglobin is affected.

Moreover, the thermal stability of fructosyl amino acid oxidase in the detection kit for detecting glycated hemoglobin on the current market is less than ideal, and a large amount of stabilizer is often added to the detection kit to improve the stability. However, diagnostic reagents require a certain period of time for storage, such that the requirement for stability is higher. In addition, the stabilizer added thereof often leads to an increase in reagent viscosity, thus affecting subsequent detection.

Therefore, a fructosyl amino acid oxidase with high substrate specificity and thermal stability is urgently needed to accurately quantify glycated hemoglobin and achieve long-term stable preservation.

### SUMMARY

The disclosure provides a recombinant protein which may have higher thermal stability and substrate specificity.

The disclosure also provides a preparation method of a recombinant protein to prepare the above-mentioned recombinant protein.

The disclosure is provided by the attached claims, wherein the preferred embodiments are shown in the dependent claims.

The disclosure further provides a detection kit for detecting glycated hemoglobin having the advantages of accurately quantifying the glycated hemoglobin and long-term stable preservation.

The disclosure provides a recombinant protein including a peptide, in which the peptide includes an amino acid sequence after substitution of a sequence as set forth in SEQ ID NO: 1. The above-mentioned substitution includes one selected from the group consisting of the following substitutions: a single substitution, in which the amino acid at position 182 of SEQ ID NO: 1 is substituted from glycine (Gly) to aspartic acid (Asp) (Gly182Asp); a double substitution, in which the amino acid at position 182 of SEQ ID NO: 1 is substituted from glycine (Gly) to aspartic acid (Asp) (Gly182Asp), and the amino acid at position 268 of SEQ ID NO: 1 is substituted from asparagine (Asn) to aspartic acid (Asp) (Asn268Asp); and a triple substitution, in which the amino acid at position 182 of SEQ ID NO: 1 is substituted from glycine (Gly) to aspartic acid (Asp) (Gly182Asp), the amino acid at position 268 of SEQ ID NO: 1 is substituted from asparagine (Asn) to aspartic acid (Asp) (Asn268Asp), and the amino acid at position 384 of SEQ ID NO: 1 is substituted from histidine (His) to tyrosine (Tyr) (His384Tyr).

In an embodiment of the disclosure, the amino acid sequence of the peptide may include a sequence as set forth in SEQ ID NO: 2.

In an embodiment of the disclosure, the amino acid sequence of the peptide may include a sequence as set forth in SEQ ID NO: 3.

In an embodiment of the disclosure, the amino acid sequence of the peptide may include a sequence as set forth in SEQ ID NO: 4.

In an embodiment of the disclosure, the amino acid sequence of SEQ ID NO: 1 and a polynucleotide encoding the sequence of SEQ ID NO: 1 may be selected from *Phaeosphaeria nodorum.*

The disclosure further provides a preparation method of a recombinant protein, including the following steps: substituting a nucleotide sequence which encodes the amino acid sequence as set forth in SEQ ID NO: 1 in a plasmid. In which, the substitution includes one selected from the group consisting of the following substitutions: a single substitution, in which nucleotides encoding the amino acid at position 182 are substituted from nucleotides encoding glycine (Gly) to nucleotides encoding aspartic acid (Asp); a double substitution, in which the nucleotides encoding the amino acid at position 182 are substituted from the nucleotides encoding glycine (Gly) to the nucleotides encoding aspartic acid (Asp), and nucleotides encoding the amino acid at position 268 are substituted from nucleotides encoding asparagine (Asn) to the nucleotides encoding aspartic acid (Asp); a triple substitution, in which the nucleotides encoding the amino acid at position 182 are substituted from the nucleotides encoding glycine (Gly) to the nucleotides encoding aspartic acid (Asp), the nucleotides encoding the amino acid at position 268 are substituted from the nucleotides encoding asparagine (Asn) to the nucleotides encoding aspartic acid (Asp), and nucleotides encoding the amino acid at position 384 are substituted from nucleotides encoding histidine (His) to nucleotides encoding tyrosine (Tyr). Next, the substituted plasmid is transformed into a host cell to obtain a transformant, and then the above-mentioned recombinant protein is expressed by the transformant.

In an embodiment of the disclosure, the step of substituting the nucleotides encoding the amino acid includes: substituting the nucleotides encoding glycine (Gly) at position 182 via a primer as set forth in SEQ ID NO: 6 and a primer as set forth in SEQ ID NO: 7, substituting the nucleotides encoding asparagine (Asn) at position 268 via a primer as set forth in SEQ ID NO: 8 and a primer as set forth in a SEQ ID NO: 9, and substituting the nucleotides encoding histidine (His) at position 384 via a primer as set forth in SEQ ID NO: 10 and a primer as set forth in SEQ ID NO: 11.

In an embodiment of the disclosure, after the nucleotides encoding glycine (Gly) at position 182 are substituted, a recombinant protein containing the amino acid sequence as set forth in SEQ ID NO: 2 may be obtained.

In an embodiment of the disclosure, after the nucleotides encoding glycine (Gly) at position 182 and the nucleotides encoding asparagine (Asn) at position 268 are substituted simultaneously, a recombinant protein containing the amino acid sequence as set forth in SEQ ID NO: 3 may be obtained.

In an embodiment of the disclosure, after the nucleotides encoding glycine (Gly) at position 182, the nucleotides encoding asparagine (Asn) at position 268, and the nucleotides encoding histidine (His) at position 384 are substituted simultaneously, a recombinant protein containing the amino acid sequence as set forth in SEQ ID NO: 4 may be obtained.

In an embodiment of the disclosure, the method of preparing the above-mentioned plasmid may include: optimizing a codon of a fructosyl amino acid oxidase gene to obtain a polynucleotide as set forth in SEQ ID NO:5, and constructing the polynucleotide as set forth in SEQ ID NO:5 in a vector to obtain a plasmid encoding the amino acid sequence as set forth in SEQ ID NO: 1.

The disclosure also provides a polynucleotide encoding the above-mentioned recombinant protein.

The disclosure further provides a detection kit for detecting glycated hemoglobin, in which the detection kit includes the above-mentioned recombinant protein.

Based on the above, the disclosure provides a recombinant protein and a preparation method thereof as set forth in claim 6, in which the recombinant protein is obtained by substituting as set forth in claim 1 one amino acid of the amino acids at positions 182, 268, and 384 of the amino acid sequence as set forth in SEQ ID NO: 1. Moreover, in comparison to the wild-type fructosyl amino acid oxidase, this recombinant protein exhibits higher thermal stability and substrate specificity. Otherwise, in comparison to the commercial detection kit for detecting glycated hemoglobin, due to that the detection kit provided by the disclosure includes the above-mentioned recombinant protein, the advantages of accurately quantifying glycated hemoglobin and long-term stable preservation are provided accordingly.

Several exemplary embodiments accompanied with figures are described in detail below to further describe the disclosure in details.

### BRIEF DESCRIPTION OF THE DRAWINGS

The accompanying drawings are included to provide further understanding, and are incorporated in and constitute a part of this specification. The drawings illustrate exemplary embodiments and, together with the description, serve to explain the principles of the disclosure.

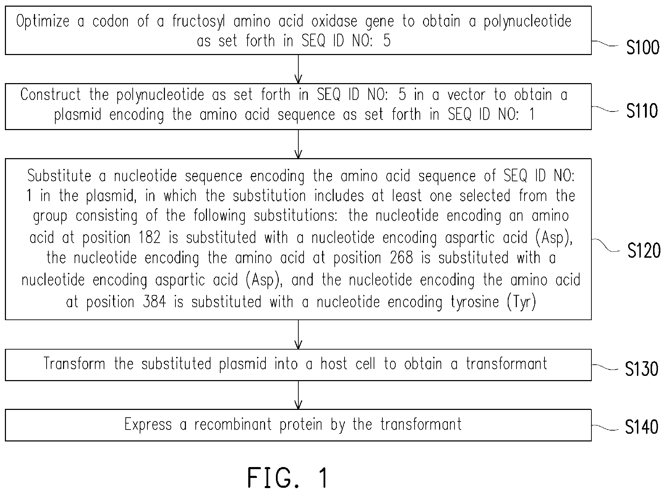

FIG. 1 illustrates the preparation process of a recombinant protein according to an embodiment of the disclosure.

### DETAILED DESCRIPTION OF DISCLOSED EMBODIMENTS

FIG. 1 is a flowchart illustrating a preparation method of a recombinant protein according to an embodiment of the disclosure. First, referring to FIG. 1, as shown in step S100, codon optimization may be performed on a fructosyl amino acid oxidase gene to obtain the polynucleotide as set forth in SEQ ID NO: 5. The above-mentioned fructosyl amino acid oxidase gene may be selected from bacteria, yeast, or fungus, such as selected from *Phaeosphaeria nodorum.* For example, it may also be selected from *Aspergillus, Penicillium, Fusarium, Pichia, Coniochaeta, Eupencillum*, or *Corynebacterium.* In an embodiment, the above-mentioned fructosyl amino acid oxidase gene may be selected from *Phaeosphaeria nodorum.* In particular, the method of codon optimization includes adjusting the nucleotide in the codon, such as adjusting GGT to GGC such that the adjusted codon is suitable for being utilized in a new host cell and an amino acid may be expressed in large quantities. Moreover, this amino acid is the same as the amino acid expressed by the codon before adjustment.

The above-mentioned fructosyl amino acid oxidase may also be referred to as, for instance, fructosyl valine oxidase, fructosyl peptide oxidase (FPOX), amadoriase or ketoamine oxidase.

Next, as shown in step S110, the polynucleotide as set forth in SEQ ID NO: 5 may be constructed in a vector to obtain a plasmid encoding the amino acid sequence as set forth in SEQ ID NO: 1.

Thereafter, as shown in step S120, the nucleotide sequence encoding the amino acid sequence of SEQ ID NO: 1 in the plasmid may be substituted, in which the substitution may include one selected from the group consisting of the following substitutions: a single substitution, in which the nucleotides encoding the amino acid at position 182 are substituted from nucleotides encoding glycine (Gly) tonucleotides encoding aspartic acid (Asp), a double substitution, in which the nucleotides encoding the amino acid at position 182 are substituted from nucleotides encoding glycine (Gly) to the nucleotides encoding aspartic acid (Asp), and the nucleotides encoding the amino acid at position 268 are substituted from nucleotides encoding asparagine (Asn) to nucleotides encoding aspartic acid (Asp), and a triple substitution, in which the nucleotides encoding the amino acid at position 182 are substituted from the nucleotides encoding glycine (Gly) to the nucleotides encoding aspartic acid (Asp), the nucleotides encoding the amino acid at position 268 are substituted from the nucleotides encoding asparagine (Asn) to the nucleotides encoding aspartic acid (Asp), and the nucleotides encoding the amino acid at position 384 are substituted from nucleotides encoding histidine (His) to the nucleotides encoding tyrosine (Tyr).

In detail, the substitution may include one selected from the group consisting of the following substitutions: a single substitution, in which the nucleotides encoding the amino acid at position 182 of SEQ ID NO: 1 are substituted from the nucleotides encoding glycine (Gly) to the nucleotides encoding aspartic acid (Asp) (Gly182Asp); a double substitution, in which the nucleotides encoding the amino acid at position 182 of SEQ ID NO: 1 are substituted from the nucleotides encoding glycine (Gly) to the nucleotides encoding aspartic acid (Asp) (Gly182Asp), and the nucleotides encoding the amino acid at position 268 of SEQ ID NO: 1 are substituted from the nucleotides encoding asparagine (Asn) to the nucleotides encoding aspartic acid (Asp) (Asn268Asp); a triple substitution, in which the nucleotides encoding the amino acid at position 182 of SEQ ID NO: 1 are substituted from the nucleotides encoding glycine (Gly) to the nucleotides encoding aspartic acid (Asp) (Gly182Asp), the nucleotides encoding the amino acid at position 268 of SEQ ID NO: 1 are substituted from the nucleotides encoding asparagine (Asn) to the nucleotides encoding aspartic acid (Asp) (Asn268Asp), and the nucleotides encoding the amino acid at position 384 of SEQ ID NO: 1 are substituted from the nucleotides encoding histidine (His) to the nucleotides encoding tyrosine (Tyr) (His384Tyr).

More specifically, the step of substituting the amino acid may include: substituting the nucleotides encoding glycine (Gly) at position 182 via a primer as set forth in SEQ ID NO: 6 and a primer as set forth in SEQ ID NO: 7, substituting the nucleotides encoding asparagine (Asn) at position 268 via a primer as set forth in SEQ ID NO: 8 and a primer as set forth in SEQ ID NO: 9, and substituting the nucleotides encoding histidine (His) at position 384 via a primer as set forth in SEQ ID NO: 10 and a primer as set forth in SEQ ID NO: 11.

Next, as shown in step S130, transformation may be performed on the above-mentioned substituted plasmid into a host cell to obtain a transformant.

Thereafter, as shown in step S140, expression a recombinant protein by the above-mentioned transformant may be performed. The above-mentioned recombinant protein may include a peptide, and the peptide may include an amino acid sequence after substitution of the sequence as set forth in the SEQ ID NO: 1. The substitution may include one selected from the group consisting of the following substitutions: a single substitution, in which nucleotides encoding the amino acid at position 182 of SEQ ID NO: 1 are substituted from nucleotides encoding glycine (Gly) to nucleotides encoding aspartic acid (Asp) (Gly182Asp); a double substitution, in which the nucleotides encoding the amino acid at position 182 of SEQ ID NO: 1 are substituted from glycine (Gly) to nucleotides encoding aspartic acid (Asp) (Gly182Asp), and nucleotides the amino acid at position 268 of SEQ ID NO: 1 are substituted from asparagine (Asn) to nucleotides encoding aspartic acid (Asp) (Asn268Asp); a triple substitution, in which the nucleotides encoding the amino acid at position 182 of SEQ ID NO: 1 are substituted from nucleotides encoding glycine (Gly) to the nucleotides encoding aspartic acid (Asp) (Gly182Asp), the nucleotides encoding the amino acid at position 268 of SEQ ID NO: 1 are substituted from the nucleotides encoding asparagine (Asn) to the nucleotides encoding aspartic acid (Asp) (Asn268Asp) and nucleotides encoding the amino acid at position 384 of SEQ ID NO: 1 are substituted from nucleotides encoding histidine (His) to nucleotides encoding tyrosine (Tyr) (His384Tyr).

In an embodiment, the preparation method as shown in steps S100 to S140 may be performed to obtain a recombinant protein. The recombinant protein is further described below.

In an embodiment, the recombinant protein includes a peptide, in which the peptide includes an amino acid sequence after substitution of a sequence as set forth in SEQ ID NO: 1. In an embodiment, the substitution may include substituting the glycine at position 182 of SEQ ID NO: 1 with aspartic acid (Asp). In another embodiment, the substitution may include substituting the glycine at position 182 of SEQ ID NO: 1 with aspartic acid (Asp) and simultaneously substituting the asparagine at position 268 of SEQ ID NO: 1 with aspartic acid (Asp). In yet another embodiment, the substitution may include substituting the glycine at position 182 of SEQ ID NO: 1 with aspartic acid (Asp), substituting the asparagine at position 268 of SEQ ID NO: 1 with aspartic acid (Asp) and substituting the histidine (His) at position 384 of SEQ ID NO: 1 with tyrosine (Tyr) simultaneously.

In an embodiment, the amino acid sequence as set forth in SEQ ID NO: 1 and the polynucleotide encoding the amino acid sequence as set forth in SEQ ID NO: 1 may be selected from *Phaeosphaeria nodorum.*

Specifically, in an embodiment, glycine at position 182 of the amino acid sequence as set forth in SEQ ID NO: 1 may be substituted to obtain the recombinant protein containing the amino acid sequence as set forth in SEQ ID NO: 2. In another embodiment, glycine at position 182 and asparagine (Asn) at position 268 of the amino acid sequence as set forth in SEQ ID NO: 1 may be substituted simultaneously to obtain the recombinant protein containing the amino acid sequence as set forth in SEQ ID NO: 3. In yet another embodiment, glycine at position 182, asparagine (Asn) at position 268, and histidine (His) at position 384 of the amino acid sequence as set forth in SEQ ID NO: 1 may be substituted simultaneously to obtain the recombinant protein containing the amino acid sequence as set forth in SEQ ID NO: 4.

In an embodiment, the above-mentioned recombinant protein may be fructosyl amino acid oxidase with oxidase activity for F-K, F-V, and F-VH, and is therefore suitable for detecting glycated hemoglobin. In another embodiment, in comparison to wild-type fructosyl amino acid oxidase, the recombinant protein containing the amino acid sequence as set forth in SEQ ID NO: 4 may have lower substrate specificity for F-K but higher substrate specificity for F-V and F-VH.

Moreover, in other embodiments, a polynucleotide, recombinant vector, transformant, and detection kit are further provided. The above-mentioned polynucleotide may be a polynucleotide encoding the above-mentioned recombinant protein, in which the polynucleotide may be a polynucleotide containing the nucleotide sequence as set forth in SEQ ID NO: 5. The above-mentioned recombinant vector may include the above-mentioned polynucleotide. The above-mentioned transformant may be obtained by transforming the above-mentioned recombinant vector into a host cell. The above-mentioned detection kit is suitable for detecting glycated hemoglobin and includes the above-mentioned recombinant protein.

### Experiments

### Experiment 1: Synthesis of fructosyl amino acid oxidase gene

First, a gene expected to encode fructosyl amino acid oxidase of *Phaeosphaeria nodorum* is selected from the genome database (http://www.ncbi.nlm.nih.gov/) of the National Center for Biotechnology Information (NCBI), such as a gene encoding a FAD-dependent oxidoreductase, and the results are similar to the mRNA shown in NCBI accession No. XM_001798659.1 (NCBI access number XM_001798659.1).

Next, for the cDNA corresponding to the above-mentioned mRNA, the cutting sites required for replication were designed at both ends thereof, and a codon thereof was optimized to a codon suitable to be expressed in a host cell. For instance, the codon was optimized to be suitable to be expressed in *E. coli*, and then the whole-genome synthesis was performed.

Next, the synthetic gene was sub-replicated in a vector, such as the EcoRV site of pUC57 (Genescript), and sequence analysis was performed on the cDNA portion replicated in the vector pUC57 to obtain the polynucleotide as set forth in SEQ ID NO: 5. In other words, the cDNA portion of the synthetic gene is the polynucleotide as set forth in SEQ ID NO: 5. The cDNA encoding region in the polynucleotide as set forth in SEQ ID NO: 5 was ranging from nucleotides NO.1 to nucleotides NO. 1314, in which the start codon was corresponding to nucleotides NO. 1 to nucleotides NO. 3, and the stop codon TAG was corresponding to nucleotides NO. 1312 to nucleotides NO. 1314.

### Experiment 2: Preparation of plasmid containing the fructosyl amino acid oxidase gene

The polynucleotide as set forth in SEQ ID NO: 5 was constructed into a vector by a cloning method, in which the vector is, for instance, pQE-30 (Qiagen).

Specifically, the polynucleotide as set forth in SEQ ID NO: 5 was replicated to the NcoI-SacI site of the vector pQE-30 via T4 DNA ligase by the NcoI restriction site introduced at the N-terminal during whole-genome synthesis and the SacI restriction site after the C-terminal stop codon to obtain the plasmid pYC1 expressing the amino acid sequence as set forth in SEQ ID NO: 1.

### Experiment 3: Performing site-directed mutagenesis on fructosyl amino acid oxidase

The above-mentioned site-directed mutagenesis refers to substituting one of the amino acid residues of FAOD, such as substituting glycine (Gly) at position 182 of the amino acid sequence as set forth in SEQ ID NO: 1 to obtain a single-mutant fructosyl amino acid oxidase. Specifically, for instance, glycine at position 182 was substituted with aspartic acid (Asp). The method of substituting the amino acid residues includes, for instance, using a QuikChange Lightning Site-Directed Mutagenesis Kit (Agilent).

In particular, the polynucleotide encoding the amino acid sequence as set forth in SEQ ID NO: 1 is SEQ ID NO: 5. Accordingly, in an embodiment, firstly, the nucleotides encoding glycine (Gly) at position 182 of the amino acid sequence as set forth in SEQ ID NO: 1 were found to be ggc in the polynucleotide as set forth in SEQ ID NO: 5. At the same time, the primer as set forth in SEQ ID NO: 6 and the primer as set forth in SEQ ID NO: 7 were synthesized, in which both primers have the nucleotides gac. Next, a polymerase chain reaction (PCR) was performed on the plasmid pYC1 using the primer as set forth in SEQ ID NO: 6 and the primer as set forth in SEQ ID NO: 7 to obtain a recombinant plasmid. In the recombinant plasmid, the nucleotides ggc encoding glycine (Gly) at position 182 were substituted by the nucleotides gac encoding aspartic acid (Asp).

Thereafter, the recombinant plasmid was transformed by One Shot® Mach1™-T1R (Invitrogen) and 10 colonies exhibiting ampicillin resistance were selected, and then sequencing was performed on the nucleotide sequence of the 10 recombinant plasmids transformed into the 10 colonies to confirm whether the recombinant plasmids were successfully introduced to obtain a plasmid pHL35 expressing the amino acid sequence as set forth in SEQ ID NO: 2.

### Experiment 4: Performing multiple point mutations on fructosyl amino acid oxidase

The above-mentioned multiple point mutation refers to substituting two of the amino acid residues of FAOD, such as substituting glycine (Gly) at position 182 and asparagine (Asn) at position 268 of the amino acid sequence as set forth in SEQ ID NO: 1 to obtain a double-mutant fructosyl amino acid oxidase. Specifically, for instance, glycine (Gly) at position 182 was substituted with aspartic acid (Asp) and asparagine (Asn) at position 268 was substituted with aspartic acid (Asp) at the same time. The method of substituting the amino acid residues is the same as above and is not repeated herein.

In particular, firstly, the nucleotides encoding asparagine (Asp) at position 268 of the amino acid sequence as set forth in SEQ ID NO: 1 were found to be aac in the polynucleotide as set forth in SEQ ID NO: 5. At the same time, the primer as set forth in SEQ ID NO: 8 and the primer as set forth in SEQ ID NO: 9 were synthesized, in which both primers have the nucleotides gac. Next, since the nucleotides encoding the amino acid at position 182 in the plasmid pHL35 were substituted with the nucleotides gac encoding aspartic acid (Asp), a polymerase chain reaction was performed on the plasmid pHL35 using the primer as set forth in SEQ ID NO: 8 and the primer as set forth in SEQ ID NO: 9 to substitute the nucleotides aac encoding asparagine (Asn) at position 268 in the plasmid pHL35 with the nucleotides gac encoding aspartic acid (Asp) to obtain a plasmid pHL43 expressing the amino acid sequence as set forth in SEQ ID NO: 3.

### Experiment 5: Performing multiple point mutations on fructosyl amino acid oxidase

The above-mentioned multiple point mutation refers to substituting three of the amino acid residues of FAOD, such as substituting glycine (Gly) at position 182, asparagine (Asn) at position 268, and histidine (His) at position 384 of the amino acid sequence as set forth in SEQ ID NO: 1 to obtain a triple-mutant fructosyl amino acid oxidase. Specifically, for instance, glycine (Gly) at position 182 was substituted with aspartic acid (Asp), asparagine (Asn) at position 268 was substituted with aspartic acid (Asp), and histidine (His) at position 384 was substituted with tyrosine (Tyr) at the same time. The method of substituting the amino acid residues is the same as above and is not repeated herein.

In particular, firstly, the nucleotides encoding histidine (His) at position 384 of the amino acid sequence as set forth in SEQ ID NO: 1 were found to be cat in the polynucleotide shown in SEQ ID NO: 5. At the same time, the primer as set forth in SEQ ID NO: 10 and the primer as set forth in SEQ ID NO: 11 were synthesized, in which both primers have the nucleotides tat. Next, since the nucleotides encoding the amino acid at position 182 and the amino acid at position 268 in the plasmid pHL43 were both substituted with the nucleotides gac encoding aspartic acid (Asp), a polymerase chain reaction was performed on the plasmid pHL43 using the primer as set forth in SEQ ID NO: 10 and the primer as set forth in SEQ ID NO: 11 to substitute the nucleotides cat encoding histidine (His) at position 384 in the plasmid pHL43 with the nucleotides tat encoding aspartic acid (Asp) to obtain a plasmid pHL37 expressing the amino acid sequence as set forth in SEQ ID NO: 4.

In short, the plasmid pHL35 may express the amino acid sequence as set forth in SEQ ID NO: 2, and the amino acid at position 182 was substituted with aspartic acid (Asp). The plasmid pHL43 may express the amino acid sequence as set forth in SEQ ID NO: 3, and the amino acids at position 182 and position 268 were both substituted with aspartic acid (Asp). The plasmid pHL37 may express the amino acid sequence as set forth in SEQ ID NO: 4, in which the amino acids at position 182 and position 268 were both substituted with aspartic acid (Asp), and the amino acid at position 384 was substituted with tyrosine (Tyr).

### Experiment 6: Mass expression and purification of fructosyl amino acid oxidase

First, the plasmid pYC1 and the plasmids pHL35, pHL43, and pHL37 after amino acid residue substitution were respectively transformed into a host cell to obtain a transformant having ampicillin resistance. The host cell may be *E. coli*, such as BL21 Star™ (DE3) (Chemically Competent *E. coli*) (Invitrogen).

Next, a culture medium for growing a transformant was prepared. First, 500 mL of TB (Terrific Broth) culture medium was dispensed into an Erlenmeyer flask with a volume of 2 L, and high-pressure steam sterilization was performed at 121°C for 20 minutes. After the culture medium was cooled down, ampicillin was added to the culture medium after sterile filtration to achieve a final ampicillin concentration of 100 µg/mL.

Then, 2 mL of a BL21 Star™ (DE3) (pYC1) culture solution grown for 16 hours at 37°C beforehand was inoculated in the above-mentioned TB culture medium, in which the culture solution was a LB (Lysogeny broth) culture medium containing 100 µg/mL ampicillin, and then growth was performed by shaking at 225 rpm at 37°C as well as the absorbance (OD600 or A600) at 600 nm was kept tracking at the same time. When the absorbance reached 0.6, the temperature was adjusted to 20°C and isopropyl-betaβ-D-thiogalactopyranoside (IPTG) was added to achieve a final IPTG concentration of 1 mM.

Thereafter, growing was continued for 20 hours. After culture was completed, bacteria was collected by centrifuge and CelLytic™ B 2X Cell Lysis Reagent (Sigma) was added to disrupt the bacteria, and a suspension containing the disrupted bacteria was centrifuged to separate and obtain the supernatant. Next, the resulting supernatant was purified using cOmplete™ His-Tag Purification Resin (Roche) to obtain a purified target protein, and then sodium dodecyl sulfate polyacrylamide gel electrophoresis (SDS-PAGE) was used to confirm whether the purified target protein was actually a single protein.

The target protein is, for instance, fructosyl peptide oxidase of *Phaeosphaeria nodorum* (PnFPOX), i.e., fructosyl amino acid oxidase, and the resulting target protein is the above-mentioned recombinant protein, i.e., the protein for which one of glycine (Gly) at position 182, asparagine (Asn) at position 268, and histidine (His) at position 384 in the amino acid sequence as set forth in SEQ ID NO: 1 is substituted as specified supra. The substitution method of the amino acid residues is provided above and is not repeated herein.

In the above-mentioned operation, the transformant containing the plasmid pYC1 may express the amino acid sequence as set forth in SEQ ID NO: 1, i.e., wild-type fructosyl amino acid oxidase (PnFPOX for short). The transformant containing the plasmid pHL35 may express the amino acid sequence as set forth in SEQ ID NO: 2, i.e., a recombinant protein for which the amino acid at position 182 of the fructosyl amino acid oxidase was substituted with aspartic acid (Asp) (PnFPOX-SS for short). The transformant containing the plasmid pHL43 may express the amino acid sequence as set forth in SEQ ID NO: 3, i.e., a recombinant protein for which the amino acid at position 182 and the amino acid at position 268 of the fructosyl amino acid oxidase were both substituted with aspartic acid (Asp) (PnFPOX-DS for short). The transformant containing the plasmid pHL37 may express the amino acid sequence as set forth in SEQ ID NO: 4, i.e., a recombinant protein for which the amino acid at position 182 and the amino acid at position 268 of the fructosyl amino acid oxidase were both substituted with aspartic acid (Asp) and the amino acid at position 384 was substituted with tyrosine (Tyr) (PnFPOX-TS for short).

### Experiment 7: Evaluation of activity, substrate specificity, and thermal stability of fructosyl amino acid oxidase

Oxidase activity, substrate specificity, and thermal stability of the recombinant proteins (PnFPOX-SS, PnFPOX-DS, and PnFPOX-TS) were detected, and the results were compared with wild-type fructosyl amino acid oxidase (PnFPOX) and fructosyl amino acid oxidase (FPOX-CE for short) of other species (made by Kikkoman Corporation).

### Evaluation of oxidase activity

The oxidase activity for the substrate was calculated by reacting oxidase and the substrate to produce hydrogen peroxide, then the hydrogen peroxide and a coloring agent were catalyzed via hydroperoxidase and a color change was occurred therein. Accordingly, the oxidase activity can be calculated by simply detecting the change in absorbance of the coloring agent at a specific wavelength.

The detection method of oxidase activity is specifically described as follows: first, an activity assay solution was prepared with reference to the literature published by Hirokawa et al. in Biochemical and Biophysical Research Communications in 2003 (vol. 311, pages 104 to 111), i.e., a 10 mM potassium phosphate buffer solution at a pH of 8.0 containing 5 mg/L of peroxidase (TOYOBO), 100 mg/L 4-aminoantipyrine (Sigma), and 0.167g/L TOOS (Sigma). Next, measurement was performed, in which 145 µl of an activity assay solution was pre-heated at 37°C for 30 minutes, and then 5 µl of an enzyme solution diluted by an enzyme diluent (10 mM potassium phosphate buffer containing 0.15% BSA (pH of 8.0)) beforehand was added in the activity assay reagent to react. After reacting at 37°C for 5 minutes, the change in absorbance of OD555 within a unit time was measured (ΔOD experiment/min). At the same time, the enzyme solution was replaced by 5 µl of an enzyme diluent, and then the same operation as above was performed and the change in absorbance (Δ OD blank/min) was measured as a blank.

Oxidase activity was calculated via the resulting change in absorbance based on the formula below with the amount of enzyme oxidizing 1 µmole of substrate within 1 minute defined as 1 unit (U). Value of oxidase activity (U/ml) = {(ΔOD experiment/min - ΔOD blank/min) X 150 (µl) X dilution ratio} / {4.5 X 5 (µl)}. In the formula above, "150 µl" represents the total amount of liquid of the detection solution, "4.5" represents absorption coefficient in millimoles, and "5 µl" represents the liquid volume of the oxidase sample.

Next, the oxidase activity of FPOX-CE, PnFPOX, PnFPOX-SS, PnFPOX-DS, and PnFPOX-TS for different substrates was measured. The detected substrates include: fructosyl lysine (F-K), fructosyl valine (F-V), and fructosyl valyl histidine (F-VH). The detection results of oxidase activity of different recombinant proteins are shown in Table 1.

**Table 1: Oxidase activity comparison of different recombinant proteins (value of oxidase activity (unit: U/mg))**

| | FPOX-CE | PnFPOX | PnFPOX-SS | PnFPOX-DS | PnFPOX-TS |
|---|---|---|---|---|---|
| F-K | 0.82 | 0.12 | 0.29 | 0.25 | 0.05 |
| F-V | 12.69 | 15.14 | 16.43 | 16.59 | 16.03 |
| F-VH | 3.18 | 2.73 | 4.22 | 4.02 | 4.05 |

It may be known from the results of Table 1 that, in comparison to the fructosyl amino acid oxidase (FPOX-CE) of other species, the oxidase activity of wild-type fructosyl amino acid oxidase (PnFPOX) for fructosyl lysine (F-K) is lower and the oxidase activity thereof for fructosyl valine (F-V) is higher. Moreover, in comparison to wild-type fructosyl amino acid oxidase (PnFPOX), the oxidase activity of triple-mutant fructosyl amino acid oxidase (PnFPOX-TS) for F-K is lower, but the oxidase activity thereof for fructosyl valine (F-V) and fructosyl valyl histidine (F-VH) is higher.

### Evaluation of substrate specificity

The substrate specificity of FPOX-CE, PnFPOX, PnFPOX-SS, PnFPOX-DS, and PnFPOX-TS for different substrates is represented by the activity ratio calculated by the following formula based on the above measured result of oxidase activity. In particular, (F-K/F-V) = (value of oxidase activity with F-K as the substrate) / (value of oxidase activity with F-V as the substrate), and a lower activity ratio calculated from the formula indicates that the protein exhibits higher substrate specificity for F-V. In addition, (F-K/F-VH) = (value of oxidase activity with F-K as the substrate) / (value of oxidase activity with F-VH as the substrate), and a lower activity ratio calculated from the formula indicates the protein exhibits higher substrate specificity for F-VH. The analysis results of substrate specificity of different recombinant proteins are shown in Table 2.

**Table 2: Substrate specificity comparison of different recombinant proteins**

| | FPOX-CE | PnFPOX | PnFPOX-SS | PnFPOX-DS | PnFPOX-TS |
|---|---|---|---|---|---|
| F-K/F-V | 6.4% | 0.8% | 1.8% | 1.5% | 0.3% |
| F-K/ F-VH | 25.6% | 4.3% | 6.9% | 6.3% | 1.1% |

It may be known from the results of Table 2 that, in comparison to the fructosyl amino acid oxidase (FPOX-CE) of other species, both the activity ratios of F-K/F-V and F-K/F-VH of wild-type fructosyl amino acid oxidase (PnFPOX) are lower, indicating the substrate specificity of PnFPOX for F-V and F-VH is higher. However, both the activity ratios of F-K/F-V and F-K/F-VH of triple-mutant fructosyl amino acid oxidase (PnFPOX-TS) are significantly lower than fructosyl amino acid oxidase (FPOX-CE) of other species, indicating the substrate specificity of PnFPOX-TS for F-V and F-VH is significantly higher than FPOX-CE, with a difference of about 20 times or more.

In other words, for substrate specificity, although the fructosyl amino acid oxidase (FPOX-CE) of other species exhibits the more extensive substrate activity, the substrate specificity thereof is poor. On the contrary, wild-type fructosyl amino acid oxidase (PnFPOX), single-mutant fructosyl amino acid oxidase (PnFPOX-SS), double-mutant fructosyl amino acid oxidase (PnFPOX-DS), and triple-mutant fructosyl amino acid oxidase (PnFPOX-TS) exhibit improved substrate specificity, and in particular, PnFPOX-TS exhibits the best improved effect on substrate specificity.

### Evaluation of thermal stability

The fructosyl amino acid oxidase (FPOX-CE) of other species, wild-type fructosyl amino acid oxidase (PnFPOX), and the recombinant proteins (PnFPOX-SS, PnFPOX-DS, and PnFPOX-TS) were respectively dissolved in a 100 mM potassium phosphate buffer (pH value of 8.0) to achieve a concentration of 0.05 mg/ml, and after a heat treatment at 25°C to 60°C for 10 minutes, the oxidase activities of the above-mentioned fructosyl amino acid oxidases and recombinant proteins were measured. At the same time, thermal stability (%) was calculated based on the following formula: thermal stability = (value of oxidase activity after heat treatment) / (value of oxidase activity without heat treatment) x 100%.

**Table 3: Thermal stability comparison of different recombinant proteins**

| | FPOX-CE | PnFPOX | PnFPOX-SS | PnFPOX-DS | PnFPOX-TS |
|---|---|---|---|---|---|
| 25°C | 100% | 100% | 100% | 100% | 100% |
| 30°C | 99% | 99% | 100% | 101% | 102% |
| 37°C | 97% | 99% | 100% | 100% | 103% |
| 40°C | 97% | 99% | 99% | 100% | 102% |
| 45°C | 87% | 100% | 99% | 102% | 104% |
| 50°C | 30% | 86% | 96% | 101% | 102% |
| 55°C | 5% | 6% | 11% | 67% | 33% |
| 60°C | 0% | 0% | 0% | 0% | 0% |

It may be known from the results of Table 3 that, in comparison to the fructosyl amino acid oxidase of other species (FPOX-CE) and wild-type fructosyl amino acid oxidase (PnFPOX), the fructosyl amino acid oxidases produced by site-directed mutagenesis or multiple point mutations, whether single-mutant, double-mutant, or triple-mutant fructosyl amino acid oxidase (corresponding to mutants PnFPOX-SS, PnFPOX-DS, and PnFPOX-TS, respectively), all three mutants retain 95% oxidase activity after a heat treatment at 50°C. When the temperature of the heat treatment reaches 55°C, the oxidase activity of both FPOX-CE and PnFPOX is reduced to less than 10%, while the oxidase activity of PnFPOX-DS and PnFPOX-TS is still retained the level of 67% and 33%, respectively. That is, in comparison to the fructosyl amino acid oxidase of other species and wild-type fructosyl amino acid oxidase (FPOX-CE and PnFPOX, respectively), the fructosyl amino acid oxidases after site-directed mutagenesis or multiple point mutations (PnFPOX-SS, PnFPOX-DS, and PnFPOX-TS) may exhibit higher thermal stability.

Based on the above, the disclosure provides a recombinant protein and a preparation method thereof as specified in claim 6, in which the recombinant protein is obtained by substituting one amino acid of the amino acids at positions 182, 268, and 384 of the amino acid sequence as set forth in SEQ ID NO: 1 as specified in claim 1. Moreover, in comparison to the wild-type fructosyl amino acid oxidase, this recombinant protein exhibits higher thermal stability and substrate specificity. Otherwise, in comparison to the commercial detection kit for detecting glycated hemoglobin, due to that the detection kit provided by the disclosure includes the above-mentioned recombinant protein, the advantages of accurately quantifying glycated hemoglobin and long-term stable preservation are provided accordingly.

## Claims

1. A recombinant protein, comprising:
a peptide, wherein the peptide comprises an amino acid sequence after substitution of a sequence as set forth in SEQ ID NO: 1, and wherein the substitution comprises one selected from a group consisting of the following substitutions:
a single substitution, in which an amino acid at position 182 of SEQ ID NO: 1 is substituted from glycine (Gly) to aspartic acid (Asp) (Gly182Asp);
a double substitution, in which the amino acid at position 182 of SEQ ID NO: 1 is substituted from glycine (Gly) to aspartic acid (Asp) (Gly182Asp), and an amino acid at position 268 of SEQ ID NO: 1 is substituted from asparagine (Asn) to aspartic acid (Asp) (Asn268Asp); and
a triple substitution, in which the amino acid at position 182 of SEQ ID NO: 1 is substituted from glycine (Gly) to aspartic acid (Asp) (Gly182Asp), the amino acid at position 268 of SEQ ID NO: 1 is substituted from asparagine (Asn) to aspartic acid (Asp) (Asn268Asp), and an amino acid at position 384 of SEQ ID NO: 1 is substituted from histidine (His) to tyrosine (Tyr) (His384Tyr).

2. The recombinant protein as claimed in claim 1, wherein the amino acid sequence of the peptide comprises a sequence as set forth in SEQ ID NO: 2.

3. The recombinant protein as claimed in claim 1, wherein the amino acid sequence of the peptide comprises a sequence as set forth in SEQ ID NO: 3.

4. The recombinant protein as claimed in claim 1, wherein the amino acid sequence of the peptide comprises a sequence as set forth in SEQ ID NO: 4.

5. The recombinant protein as claimed in claim 1, wherein the amino acid sequence of SEQ ID NO: 1 and a polynucleotide encoding the sequence of SEQ ID NO: 1 are selected from *Phaeosphaeria nodorum.*

6. A preparation method of a recombinant protein, comprising the following steps:
substituting a nucleotide sequence encoding an amino acid sequence as set forth in SEQ ID NO: 1 in a plasmid, wherein the substitution comprises one selected from a group consisting of the following substitutions:
a single substitution, in which nucleotides encoding an amino acid at position 182 are substituted from nucleotides encoding glycine (Gly) to nucleotides encoding aspartic acid (Asp);
a double substitution, in which the nucleotides encoding the amino acid at position 182 are substituted from the nucleotides encoding glycine (Gly) to the nucleotides encoding aspartic acid (Asp), and nucleotides encoding an amino acid at position 268 are substituted from nucleotides encoding asparagine (Asn) to nucleotides encoding aspartic acid (Asp); and
a triple substitution, in which the nucleotides encoding the amino acid at position 182 are substituted from the nucleotides encoding glycine (Gly) to the nucleotides encoding aspartic acid (Asp), the nucleotides encoding the amino acid at position 268 are substituted from the nucleotides encoding asparagine (Asn) to the nucleotides encoding aspartic acid (Asp), and nucleotides encoding an amino acid at position 384 are substituted from nucleotides encoding histidine (His) to nucleotides encoding tyrosine (Tyr);
transforming the substituted plasmid into a host cell to obtain a transformant; and
expressing the recombinant protein by the transformant.

7. The preparation method of the recombinant protein as claimed in claim 6, wherein the step of substituting the nucleotide encoding the amino acid comprises:
substituting the nucleotides encoding glycine (Gly) at position 182 via a primer as set forth in SEQ ID NO: 6 and a primer as set forth in SEQ ID NO: 7;
substituting the nucleotides encoding asparagine (Asn) at position 268 via a primer as set forth in SEQ ID NO: 8 and a primer as set forth in SEQ ID NO: 9; and
substituting the nucleotides encoding histidine (His) at position 384 via a primer as set forth in SEQ ID NO: 10 and a primer as set forth in SEQ ID NO: 11.

8. The preparation method of the recombinant protein as claimed in claim 6, wherein the nucleotides encoding glycine (Gly) at position 182 are substituted to obtain the recombinant protein containing the amino acid sequence as set forth in SEQ ID NO: 2.

9. The preparation method of the recombinant protein as claimed in claim 6, wherein the nucleotides encoding glycine (Gly) at position 182 and the nucleotides encoding asparagine (Asn) at position 268 are substituted simultaneously to obtain the recombinant protein containing the amino acid sequence as set forth in SEQ ID NO: 3.

10. The preparation method of the recombinant protein as claimed in claim 6, wherein the nucleotides encoding glycine (Gly) at position 182, the nucleotides encoding asparagine (Asn) at position 268, and the nucleotides encoding histidine (His) at position 384 are substituted simultaneously to obtain the recombinant protein containing the amino acid sequence as set forth in SEQ ID NO: 4.

11. The preparation method of the recombinant protein as claimed in claim 6, wherein the plasmid is prepared by a method of:
optimizing a codon of a fructosyl amino acid oxidase gene to obtain a polynucleotide as set forth in SEQ ID NO: 5; and
constructing the polynucleotide as set forth in SEQ ID NO: 5 in a vector to obtain the plasmid encoding the amino acid sequence as set forth in SEQ ID NO: 1.

12. A polynucleotide encoding the recombinant protein as claimed in claim 1.

13. A detection kit for detecting a glycated hemoglobin, comprising the recombinant protein as claimed in claim 1.

## Patentansprüche

1. Rekombinantes Protein, umfassend:
ein Peptid, wobei das Peptid eine Aminosäuresequenz nach Substitution einer Sequenz, wie in SEQ ID NO: 1 dargelegt, umfasst, und wobei die Substitution eine ausgewählt aus einer Gruppe, bestehend aus den folgenden Substitutionen, umfasst:
eine Einzelsubstitution, bei der eine Aminosäure an Position 182 von SEQ ID NO: 1 von Glycin (Gly) zu Asparaginsäure (Asp) (Gly182Asp) substituiert ist;
eine Doppelsubstitution, bei der die Aminosäure an Position 182 von SEQ ID NO: 1 von Glycin (Gly) zu Asparaginsäure (Asp) (Gly182Asp) substituiert ist und eine Aminosäure an Position 268 von SEQ ID NO: 1 von Asparagin (Asn) zu Asparaginsäure (Asp) (Asn268Asp) substituiert ist; und
eine Dreifachsubstitution, bei der die Aminosäure an Position 182 von SEQ ID NO: 1 von Glycin (Gly) zu Asparaginsäure (Asp) (Gly182Asp) substituiert ist, die Aminosäure an Position 268 von SEQ ID NO: 1 von Asparagin (Asn) zu Asparaginsäure (Asp) (Asn268Asp) substituiert ist und eine Aminosäure an Position 384 von SEQ ID NO: 1 von Histidin (His) zu Tyrosin (Tyr) (His384Tyr) substituiert ist.

2. Rekombinantes Protein gemäß Anspruch 1, wobei die Aminosäuresequenz des Peptids eine Sequenz, wie in SEQ ID NO: 2 dargelegt, umfasst.

3. Rekombinantes Protein gemäß Anspruch 1, wobei die Aminosäuresequenz des Peptids eine Sequenz, wie in SEQ ID NO: 3 dargelegt, umfasst.

4. Rekombinantes Protein gemäß Anspruch 1, wobei die Aminosäuresequenz des Peptids eine Sequenz, wie in SEQ ID NO: 4 dargelegt, umfasst.

5. Rekombinantes Protein gemäß Anspruch 1, wobei die Aminosäuresequenz von SEQ ID NO: 1 und ein Polynukleotid, das für die Sequenz von SEQ ID NO: 1 kodiert, ausgewählt sind aus *Phaeosphaeria nodorum.*

6. Herstellungsverfahren für ein rekombinantes Protein, umfassend die folgenden Schritte:
Substituieren einer Nukleotidsequenz, die eine Aminosäuresequenz codiert, wie in SEQ ID NO: 1 dargelegt, in einem Plasmid, wobei die Substitution eine umfasst, ausgewählt aus einer Gruppe, bestehend aus den folgenden Substitutionen:
eine Einfachsubstitution, bei der Nukleotide, kodierend eine Aminosäure an Position 182, von Nukleotiden, kodierend Glycin (Gly), zu Nukleotiden, kodierend Asparaginsäure (Asp), substituiert sind;
eine Doppelsubstitution, bei der die Nukleotide, kodierend die Aminosäure an Position 182, von den Nukleotiden, kodierend Glycin (Gly), zu den Nukleotiden, kodierend Asparaginsäure (Asp), substituiert sind, und Nukleotide, kodierend eine Aminosäure an Position 268, von Nukleotiden, kodierend Asparagin (Asn), zu Nukleotiden, kodierend Asparaginsäure (Asp), substituiert sind; und
eine Dreifachsubstitution, bei der die Nukleotiden, kodierend die Aminosäure an Position 182, von den Nukleotiden, kodierend Glycin (Gly), zu den Nukleotiden, kodierend Asparaginsäure (Asp), substituiert sind, die Nukleotide, kodierend die Aminosäure in Position 268, von den Nukleotiden, kodierend Asparagin (Asn), zu den Nukleotiden, kodierend Asparaginsäure (Asp), substituiert sind, und Nukleotide, kodierend eine Aminosäure an Position 384, von Nukleotiden, kodierend Histidin (His), zu Nukleotiden, kodierend Tyrosin (Tyr), substituiert sind;
Transformieren des substituierten Plasmids in eine Wirtszelle, um einen Transformanten zu erhalten; und
Exprimieren des rekombinanten Proteins durch den Transformanten.

7. Herstellungsverfahren des rekombinanten Proteins gemäß Anspruch 6, wobei der Schritt des Substituierens des für die Aminosäure kodierenden Nukleotids umfasst:
Substituieren der Nukleotide, kodierend Glycin (Gly) an Position 182, über einen Primer, wie in SEQ ID NO: 6 dargelegt, und einen Primer, wie in SEQ ID NO: 7 dargelegt;
Substituieren der Nukleotide, kodierend Asparagin (Asn) an Position 268, über einen Primer, wie in SEQ ID NO: 8 dargelegt, und einen Primer, wie in SEQ ID NO: 9 dargelegt; und
Substituieren der Nukleotide, kodierend Histidin (His) an Position 384, über einen Primer, wie in SEQ ID NO: 10 dargelegt, und einen Primer, wie in SEQ ID NO: 11 dargelegt.

8. Herstellungsverfahren des rekombinanten Proteins gemäß Anspruch 6, wobei die für Glycin (Gly) an Position 182 kodierenden Nukleotide substituiert sind, um das rekombinante Protein zu erhalten, das die in SEQ ID NO: 2 dargelegte Aminosäuresequenz enthält.

9. Herstellungsverfahren des rekombinanten Proteins gemäß Anspruch 6, wobei die Nukleotide, kodierend Glycin (Gly) an Position 182 und die Nukleotide, kodierend Asparagin (Asn) an Position 268 gleichzeitig substituiert sind, um das rekombinante Protein zu erhalten, das die Aminosäuresequenz , die in SEQ ID NO: 3 dargelegt ist, enthält.

10. Herstellungsverfahren des rekombinanten Proteins gemäß Anspruch 6, wobei die Nukleotide, kodierend Glycin (Gly) an Position 182, die Nukleotide, kodierend Asparagin (Asn) an Position 268, und die Nukleotide, kodierend Histidin (His) an Position 384, gleichzeitig substituiert sind, um das rekombinante Protein zu erhalten, das die Aminosäuresequenz , wie in SEQ ID NO: 4 dargelegt, enthält.

11. Herstellungsverfahren des rekombinanten Proteins gemäß Anspruch 6, wobei das Plasmid hergestellt wird nach einem Verfahren von:
Optimieren eines Codons eines Fructosylaminosäure-Oxidase-Gens, um ein Polynukleotid zu erhalten, wie in SEQ ID NO: 5 dargelegt; und
Konstruieren des Polynukleotids, wie in SEQ ID NO: 5 dargelegt, in einem Vektor, um das Plasmid zu erhalten, kodierend die in SEQ ID NO: 1 dargestellte Aminosäuresequenz.

12. Polynukleotid, kodierend das rekombinante Protein gemäß Anspruch 1.

13. Detektionskit zum Detektieren eines glykierten Hämoglobins, umfassend das rekombinante Protein gemäß Anspruch 1.

## Revendications

1. Protéine recombinante, comprenant :
un peptide, dans laquelle le peptide comprend une séquence d'acides aminés après la substitution d'une séquence telle que présentée dans SEQ ID NO : 1, et dans laquelle la substitution comprend au moins une sélectionnée dans un groupe consistant en les substitutions suivantes :
une substitution simple, dans laquelle l'acide aminé en position 182 de SEQ ID NO : 1 connait une substitution de l'acide aspartique (Asp) à la glycine (Gly) (Gly182Asp) ;
une substitution double, dans laquelle l'acide aminé en position 182 de SEQ ID NO : 1 connait une substitution de l'acide aspartique (Asp) à la glycine (Gly) (Gly182Asp), et un acide aminé en position 268 de SEQ ID NO : 1 connaît une substitution de l'acide aspartique (Asp) à l'asparagine (Asn) (Asn268Asp) ; et
une substitution triple, dans laquelle l'acide aminé en position 182 de SEQ ID NO : 1 connait une substitution de l'acide aspartique (Asp) à la glycine (Gly) (Gly182Asp), l'acide aminé en position 268 de SEQ ID NO : 1 connaît une substitution de l'acide aspartique (Asp) à l'asparagine (Asn) (Asn268Asp), et un acide aminé en position 384 de SEQ ID NO : 1 connaît une substitution de la tyrosine (Tyr) à l'histidine (His) (His384Tyr).

2. Protéine recombinante selon la revendication 1, dans laquelle la séquence d'acides aminés du peptide comprend une séquence telle qu'exposée dans SEQ ID NO : 2.

3. Protéine recombinante selon la revendication 1, dans laquelle la séquence d'acides aminés du peptide comprend une séquence telle qu'exposée dans SEQ ID NO : 3.

4. Protéine recombinante selon la revendication 1, dans laquelle la séquence d'acides aminés du peptide comprend une séquence telle qu'exposée dans SEQ ID NO : 4.

5. Protéine recombinante selon la revendication 1, dans laquelle la séquence d'acides aminés de SEQ ID NO : 1 et un polynucléotide codant pour la séquence de SEQ ID NO : 1 sont sélectionnés à partir de Phaeosphaeria nodorum.

6. Un procédé de préparation d'une protéine recombinante, comprenant les étapes suivantes :
substitution d'une séquence nucléotidique codant pour une séquence d'acides aminés telle qu'exposée dans SEQ ID NO : 1 dans un plasmide, dans lequel la substitution comprend au moins une sélectionnée dans un groupe consistant en les substitutions suivantes :
une substitution simple, dans laquelle les nucléotides codant pour un acide aminé en position 182 connaissent une substitution de nucléotides codant pour l'acide aspartique (Asp) aux nucléotides codant pour la glycine (Gly) ;
une substitution double, dans laquelle les nucléotides codant pour l'acide aminé en position 182 connaissent une substitution de nucléotides codant pour l'acide aspartique (Asp) aux nucléotides codant pour la glycine (Gly), et les nucléotides codant pour un acide aminé en position 268 connaissent une substitution de nucléotides codant pour l'acide aspartique (Asp) aux nucléotides codant pour l'asparagine (Asn) ; et
une substitution triple, dans laquelle les nucléotides codant pour l'acide aminé en position 182 connaissent une substitution de nucléotides codant pour l'acide aspartique (Asp) aux nucléotides codant pour la glycine (Gly), les nucléotides codant pour l'acide aminé en position 268 connaissent une substitution de nucléotides codant pour l'acide aspartique (Asp) aux nucléotides codant pour l'asparagine (Asn), et les nucléotides codant pour un acide aminé en position 384 connaissent une substitution de nucléotides codant pour la tyrosine (Tyr) aux nucléotides codant pour l'histidine (His) ;
transformation du plasmide substitué dans une cellule hôte pour obtenir un transformant ; et
expression de la protéine recombinante par le transformant.

7. Procédé de préparation de la protéine recombinante selon la revendication 6, dans lequel l'étape de substitution du nucléotide codant pour l'acide aminé comprend :
la substitution des nucléotides codant pour la glycine (Gly) en position 182 via une amorce telle qu'exposée dans SEQ ID NO : 6 et une amorce telle qu'exposée dans SEQ ID NO : 7 ;
la substitution des nucléotides codant pour l'asparagine (Asn) en position 268 via une amorce telle qu'exposée dans SEQ ID NO : 8 et une amorce telle qu'exposée dans SEQ ID NO : 9 ; et
la substitution des nucléotides codant pour l'histidine (His) en position 384 via une amorce telle qu'exposée dans SEQ ID NO : 10 et une amorce telle qu'exposée dans SEQ ID NO : 11.

8. Procédé de préparation de la protéine recombinante selon la revendication 6, dans lequel les nucléotides codant pour la glycine (Gly) en position 182 sont substitués pour obtenir la protéine recombinante contenant la séquence d'acides aminés telle qu'exposée dans SEQ ID NO : 2.

9. Procédé de préparation de la protéine recombinante selon la revendication 6, dans lequel les nucléotides codant pour la glycine (Gly) en position 182 et les nucléotides codant pour l'asparagine (Asn) en position 268 sont simultanément substitués pour obtenir la protéine recombinante contenant la séquence d'acides aminés telle qu'exposée dans SEQ ID NO : 3.

10. Procédé de préparation de la protéine recombinante selon la revendication 6, dans lequel les nucléotides codant pour la glycine (Gly) en position 182, les nucléotides codant pour l'asparagine (Asn) en position 268, et les nucléotides codant pour l'histidine (His) en position 384 sont simultanément substitués pour obtenir la protéine recombinante contenant la séquence d'acides aminés telle qu'exposée dans SEQ ID NO : 4.

11. Procédé de préparation de la protéine recombinante selon la revendication 6, dans lequel le plasmide est préparé par un procédé comprenant :
l'optimisation d'un codon d'un gène d'oxydase d'acide aminé fructosyle pour obtenir un polynucléotide tel qu'exposé dans SEQ ID NO : 5 ; et
la construction du polynucléotide tel qu'exposé dans SEQ ID NO : 5 dans un vecteur pour obtenir le plasmide codant pour la séquence d'acides aminés telle qu'exposée dans SEQ ID NO : 1.

12. Polynucléotide codant pour la protéine recombinante selon la revendication 1.

13. Kit de détection pour la détection d'une hémoglobine glyquée, comprenant la protéine recombinante selon la revendication 1.


### SEQUENCE LISTING

<110> Industrial Technology Research Institute
<120> RECOMBINANT PROTEIN, AND PREPARATION METHOD AND APPLICATION THEREOF
<130> 66204-US-PA
<150> US 62/433,733
   <151> 2016-12-13
<160> 11
<170> PatentIn version 3.5
<210> 1
   <211> 437
   <212> PRT
   <213> Phaeosphaeria nodorum
<400> 1
   Met Ala Pro Ser Arg Ala Asn Thr Ser Val Ile Val Val Gly Gly Gly
   1               5                   10                  15
   Gly Thr Ile Gly Ser Ser Thr Ala Leu His Leu Val Arg Ser Gly Tyr
               20                  25                  30
   Thr Pro Ser Asn Val Thr Val Leu Asp Ala Tyr Pro Ile Pro Ser Ser
           35                  40                  45
   Gln Ser Ala Gly Asn Asp Leu Asn Lys Ile Met Gly Val Ser Leu Arg
       50                  55                  60
   Asn Pro Val Asp Leu Gln Leu Ala Leu Glu Ala Arg Gln Met Trp Asn
   65                  70                  75                  80
   Glu Asp Glu Leu Phe Lys Lys Phe Phe His Asn Thr Gly Arg Leu Asp
                   85                  90                  95
   Cys Ala His Gly Glu Lys Asp Ile Ala Asp Leu Lys Ser Gly Tyr Gln
               100                 105                 110
   Ala Leu Val Asp Ala Gly Leu Asp Ala Thr Asn Glu Trp Leu Asp Ser
           115                 120                 125
   Glu Asp Glu Ile Leu Lys Arg Met Pro Leu Leu Ser Arg Asp Gln Ile
       130                 135                 140
   Lys Gly Trp Lys Ala Ile Phe Ser Lys Asp Gly Gly Trp Leu Ala Ala
   145                 150                 155                 160
   Ala Lys Ala Ile Asn Ala Val Gly Glu Tyr Leu Arg Asp Gln Gly Val
   165 170 175
   Arg Phe Gly Phe Tyr Gly Ala Gly Ser Phe Lys Ala Pro Leu Leu Ala
   180 185 190
   Glu Gly Val Cys Ile Gly Val Glu Thr Val Asp Gly Thr Arg Tyr Tyr
   195 200 205
   Ala Asp Lys Val Val Leu Ala Ala Gly Ala Trp Ser Pro Thr Leu Val
   210 215 220
   Glu Leu His Glu Gln Cys Val Ser Lys Ala Trp Val Tyr Gly His Ile
   225 230 235 240
   Gln Leu Thr Pro Glu Glu Ala Ala Arg Tyr Lys Asn Ser Pro Val Val
   245 250 255
   Tyr Asn Gly Asp Val Gly Phe Phe Phe Glu Pro Asn Glu His Gly Val
   260 265 270
   Ile Lys Val Cys Asp Glu Phe Pro Gly Phe Thr Arg Phe Lys Met His
   275 280 285
   Gln Pro Phe Gly Ala Lys Ala Pro Lys Arg Ile Ser Val Pro Arg Ser
   290 295 300
   His Ala Lys His Pro Thr Asp Thr Ile Pro Asp Ala Ser Asp Val Ser
   305 310 315 320
   Ile Arg Arg Ala Ile Ala Thr Phe Met Pro Gln Phe Lys Asn Lys Lys
   325 330 335
   Met Phe Asn Gln Ala Met Cys Trp Cys Thr Asp Thr Ala Asp Ala Ala
   340 345 350
   Leu Leu Ile Cys Glu His Pro Glu Trp Lys Asn Phe Val Leu Ala Thr
   355 360 365
   Gly Asp Ser Gly His Ser Phe Lys Leu Leu Pro Asn Ile Gly Lys His
   370 375 380
   Val Val Glu Leu Leu Glu Gly Thr Leu Ala Asp Asp Leu Ala His Ala
   385 390 395 400
   Trp Arg Trp Arg Pro Gly Ser Gly Asp Ala Leu Lys Ser Arg Arg Ser
   405 410 415
   Ala Pro Ala Lys Asp Leu Ala Asp Met Pro Gly Trp Asn His Asp Lys
   420 425 430
   Pro Arg Ala Asn Leu
   435
<210> 2
   <211> 437
   <212> PRT
   <213> Phaeosphaeria nodorum
<400> 2
   Met Ala Pro Ser Arg Ala Asn Thr Ser Val Ile Val Val Gly Gly Gly
   1 5 10 15
   Gly Thr Ile Gly Ser Ser Thr Ala Leu His Leu Val Arg Ser Gly Tyr
   20 25 30
   Thr Pro Ser Asn Val Thr Val Leu Asp Ala Tyr Pro Ile Pro Ser Ser
   35 40 45
   Gln Ser Ala Gly Asn Asp Leu Asn Lys Ile Met Gly Val Ser Leu Arg
   50 55 60
   Asn Pro Val Asp Leu Gln Leu Ala Leu Glu Ala Arg Gln Met Trp Asn
   65 70 75 80
   Glu Asp Glu Leu Phe Lys Lys Phe Phe His Asn Thr Gly Arg Leu Asp
   85 90 95
   Cys Ala His Gly Glu Lys Asp Ile Ala Asp Leu Lys Ser Gly Tyr Gln
   100 105 110
   Ala Leu Val Asp Ala Gly Leu Asp Ala Thr Asn Glu Trp Leu Asp Ser
   115 120 125
   Glu Asp Glu Ile Leu Lys Arg Met Pro Leu Leu Ser Arg Asp Gln Ile
   130 135 140
   Lys Gly Trp Lys Ala Ile Phe Ser Lys Asp Gly Gly Trp Leu Ala Ala
   145 150 155 160
   Ala Lys Ala Ile Asn Ala Val Gly Glu Tyr Leu Arg Asp Gln Gly Val
   165 170 175
   Arg Phe Gly Phe Tyr Asp Ala Gly Ser Phe Lys Ala Pro Leu Leu Ala
   180 185 190
   Glu Gly Val Cys Ile Gly Val Glu Thr Val Asp Gly Thr Arg Tyr Tyr
           195                 200                 205
   Ala Asp Lys Val Val Leu Ala Ala Gly Ala Trp Ser Pro Thr Leu Val
       210                 215                 220
   Glu Leu His Glu Gln Cys Val Ser Lys Ala Trp Val Tyr Gly His Ile
   225                 230                 235                 240
   Gln Leu Thr Pro Glu Glu Ala Ala Arg Tyr Lys Asn Ser Pro Val Val
                   245                 250                 255
   Tyr Asn Gly Asp Val Gly Phe Phe Phe Glu Pro Asn Glu His Gly Val
               260                 265                 270
   Ile Lys Val Cys Asp Glu Phe Pro Gly Phe Thr Arg Phe Lys Met His
           275                 280                 285
   Gln Pro Phe Gly Ala Lys Ala Pro Lys Arg Ile Ser Val Pro Arg Ser
       290                 295                 300
   His Ala Lys His Pro Thr Asp Thr Ile Pro Asp Ala Ser Asp Val Ser
   305                 310                 315                 320
   Ile Arg Arg Ala Ile Ala Thr Phe Met Pro Gln Phe Lys Asn Lys Lys
                   325                 330                 335
   Met Phe Asn Gln Ala Met Cys Trp Cys Thr Asp Thr Ala Asp Ala Ala
               340                 345                 350
   Leu Leu Ile Cys Glu His Pro Glu Trp Lys Asn Phe Val Leu Ala Thr
           355                 360                 365
   Gly Asp Ser Gly His Ser Phe Lys Leu Leu Pro Asn Ile Gly Lys His
       370                 375                 380
   Val Val Glu Leu Leu Glu Gly Thr Leu Ala Asp Asp Leu Ala His Ala
   385                 390                 395                 400
   Trp Arg Trp Arg Pro Gly Ser Gly Asp Ala Leu Lys Ser Arg Arg Ser
                   405                 410                 415
   Ala Pro Ala Lys Asp Leu Ala Asp Met Pro Gly Trp Asn His Asp Lys
               420                 425                 430
   Pro Arg Ala Asn Leu
           435
<210> 3
   <211> 437
   <212> PRT
   <213> Phaeosphaeria nodorum
<400> 3
   Met Ala Pro Ser Arg Ala Asn Thr Ser Val Ile Val Val Gly Gly Gly
   1               5                   10                  15
   Gly Thr Ile Gly Ser Ser Thr Ala Leu His Leu Val Arg Ser Gly Tyr
               20                  25                  30
   Thr Pro Ser Asn Val Thr Val Leu Asp Ala Tyr Pro Ile Pro Ser Ser
           35                  40                  45
   Gln Ser Ala Gly Asn Asp Leu Asn Lys Ile Met Gly Val Ser Leu Arg
       50                  55                  60
   Asn Pro Val Asp Leu Gln Leu Ala Leu Glu Ala Arg Gln Met Trp Asn
   65                  70                  75                  80
   Glu Asp Glu Leu Phe Lys Lys Phe Phe His Asn Thr Gly Arg Leu Asp
                   85                  90                  95
   Cys Ala His Gly Glu Lys Asp Ile Ala Asp Leu Lys Ser Gly Tyr Gln
               100                 105                 110
   Ala Leu Val Asp Ala Gly Leu Asp Ala Thr Asn Glu Trp Leu Asp Ser
           115                 120                 125
   Glu Asp Glu Ile Leu Lys Arg Met Pro Leu Leu Ser Arg Asp Gln Ile
       130                 135                 140
   Lys Gly Trp Lys Ala Ile Phe Ser Lys Asp Gly Gly Trp Leu Ala Ala
   145                 150                 155                 160
   Ala Lys Ala Ile Asn Ala Val Gly Glu Tyr Leu Arg Asp Gln Gly Val
                   165                 170                 175
   Arg Phe Gly Phe Tyr Asp Ala Gly Ser Phe Lys Ala Pro Leu Leu Ala
               180                 185                 190
   Glu Gly Val Cys Ile Gly Val Glu Thr Val Asp Gly Thr Arg Tyr Tyr
           195                 200                 205
   Ala Asp Lys Val Val Leu Ala Ala Gly Ala Trp Ser Pro Thr Leu Val
       210                 215                 220
   Glu Leu His Glu Gln Cys Val Ser Lys Ala Trp Val Tyr Gly His Ile
   225                 230                 235                 240
   Gln Leu Thr Pro Glu Glu Ala Ala Arg Tyr Lys Asn Ser Pro Val Val
                   245                 250                 255
   Tyr Asn Gly Asp Val Gly Phe Phe Phe Glu Pro Asp Glu His Gly Val
               260                 265                 270
   Ile Lys Val Cys Asp Glu Phe Pro Gly Phe Thr Arg Phe Lys Met His
           275                 280                 285
   Gln Pro Phe Gly Ala Lys Ala Pro Lys Arg Ile Ser Val Pro Arg Ser
       290                 295                 300
   His Ala Lys His Pro Thr Asp Thr Ile Pro Asp Ala Ser Asp Val Ser
   305                 310                 315                 320
   Ile Arg Arg Ala Ile Ala Thr Phe Met Pro Gln Phe Lys Asn Lys Lys
                   325                 330                 335
   Met Phe Asn Gln Ala Met Cys Trp Cys Thr Asp Thr Ala Asp Ala Ala
               340                 345                 350
   Leu Leu Ile Cys Glu His Pro Glu Trp Lys Asn Phe Val Leu Ala Thr
           355                 360                 365
   Gly Asp Ser Gly His Ser Phe Lys Leu Leu Pro Asn Ile Gly Lys His
       370                 375                 380
   Val Val Glu Leu Leu Glu Gly Thr Leu Ala Asp Asp Leu Ala His Ala
   385                 390                 395                 400
   Trp Arg Trp Arg Pro Gly Ser Gly Asp Ala Leu Lys Ser Arg Arg Ser
                   405                 410                 415
   Ala Pro Ala Lys Asp Leu Ala Asp Met Pro Gly Trp Asn His Asp Lys
               420                 425                 430
   Pro Arg Ala Asn Leu
           435
<210> 4
   <211> 437
   <212> PRT
   <213> Phaeosphaeria nodorum
<400> 4
   Met Ala Pro Ser Arg Ala Asn Thr Ser Val Ile Val Val Gly Gly Gly
   1               5                   10                  15
   Gly Thr Ile Gly Ser Ser Thr Ala Leu His Leu Val Arg Ser Gly Tyr
               20                  25                  30
   Thr Pro Ser Asn Val Thr Val Leu Asp Ala Tyr Pro Ile Pro Ser Ser
           35                  40                  45
   Gln Ser Ala Gly Asn Asp Leu Asn Lys Ile Met Gly Val Ser Leu Arg
       50                  55                  60
   Asn Pro Val Asp Leu Gln Leu Ala Leu Glu Ala Arg Gln Met Trp Asn
   65                  70                  75                  80
   Glu Asp Glu Leu Phe Lys Lys Phe Phe His Asn Thr Gly Arg Leu Asp
                   85                  90                  95
   Cys Ala His Gly Glu Lys Asp Ile Ala Asp Leu Lys Ser Gly Tyr Gln
               100                 105                 110
   Ala Leu Val Asp Ala Gly Leu Asp Ala Thr Asn Glu Trp Leu Asp Ser
           115                 120                 125
   Glu Asp Glu Ile Leu Lys Arg Met Pro Leu Leu Ser Arg Asp Gln Ile
       130                 135                 140
   Lys Gly Trp Lys Ala Ile Phe Ser Lys Asp Gly Gly Trp Leu Ala Ala
   145                 150                 155                 160
   Ala Lys Ala Ile Asn Ala Val Gly Glu Tyr Leu Arg Asp Gln Gly Val
                   165                 170                 175
   Arg Phe Gly Phe Tyr Asp Ala Gly Ser Phe Lys Ala Pro Leu Leu Ala
               180                 185                 190
   Glu Gly Val Cys Ile Gly Val Glu Thr Val Asp Gly Thr Arg Tyr Tyr
           195                 200                 205
   Ala Asp Lys Val Val Leu Ala Ala Gly Ala Trp Ser Pro Thr Leu Val
       210                 215                 220
   Glu Leu His Glu Gln Cys Val Ser Lys Ala Trp Val Tyr Gly His Ile
   225                 230                 235                 240
   Gln Leu Thr Pro Glu Glu Ala Ala Arg Tyr Lys Asn Ser Pro Val Val
                   245                 250                 255
   Tyr Asn Gly Asp Val Gly Phe Phe Phe Glu Pro Asp Glu His Gly Val
               260                 265                 270
   Ile Lys Val Cys Asp Glu Phe Pro Gly Phe Thr Arg Phe Lys Met His
           275                 280                 285
   Gln Pro Phe Gly Ala Lys Ala Pro Lys Arg Ile Ser Val Pro Arg Ser
       290                 295                 300
   His Ala Lys His Pro Thr Asp Thr Ile Pro Asp Ala Ser Asp Val Ser
   305                 310                 315                 320
   Ile Arg Arg Ala Ile Ala Thr Phe Met Pro Gln Phe Lys Asn Lys Lys
                   325                 330                 335
   Met Phe Asn Gln Ala Met Cys Trp Cys Thr Asp Thr Ala Asp Ala Ala
               340                 345                 350
   Leu Leu Ile Cys Glu His Pro Glu Trp Lys Asn Phe Val Leu Ala Thr
           355                 360                 365
   Gly Asp Ser Gly His Ser Phe Lys Leu Leu Pro Asn Ile Gly Lys Tyr
       370                 375                 380
   Val Val Glu Leu Leu Glu Gly Thr Leu Ala Asp Asp Leu Ala His Ala
   385                 390                 395                 400
   Trp Arg Trp Arg Pro Gly Ser Gly Asp Ala Leu Lys Ser Arg Arg Ser
                   405                 410                 415
   Ala Pro Ala Lys Asp Leu Ala Asp Met Pro Gly Trp Asn His Asp Lys
               420                 425                 430
   Pro Arg Ala Asn Leu
           435
<210> 5
   <211> 1314
   <212> DNA
   <213> Artificial sequence
<220>
   <223> A polynucleotide encoding the amino acid sequence shown in SEQ ID NO: 1
<220>
   <221> misc_feature
   <222> (6)..(6)
   <223> n is a, c, g, or t
<220>
   <221> misc_feature
   <222> (9)..(9)
   <223> n is a, c, g, or t
<220>
   <221> misc_feature
   <222> (12)..(12)
   <223> n is a, c, g, or t
<220>
   <221> misc_feature
   <222> (15)..(15)
   <223> n is a, c, g, or t
<220>
   <221> misc_feature
   <222> (18)..(18)
   <223> n is a, c, g, or t
<220>
   <221> misc_feature
   <222> (24)..(24)
   <223> n is a, c, g, or t
<220>
   <221> misc_feature
   <222> (27)..(27)
   <223> n is a, c, g, or t
<220>
   <221> misc_feature
   <222> (30)..(30)
   <223> n is a, c, g, or t
<220>
   <221> misc_feature
   <222> (36)..(36)
   <223> n is a, c, g, or t
<220>
   <221> misc_feature
   <222> (39)..(39)
   <223> n is a, c, g, or t
<220>
   <221> misc_feature
   <222> (42)..(42)
   <223> n is a, c, g, or t
<220>
   <221> misc_feature
   <222> (45)..(45)
   <223> n is a, c, g, or t
<220>
   <221> misc_feature
   <222> (48)..(48)
   <223> n is a, c, g, or t
<220>
   <221> misc_feature
   <222> (51)..(51)
   <223> n is a, c, g, or t
<220>
   <221> misc_feature
   <222> (54)..(54)
   <223> n is a, c, g, or t
<220>
   <221> misc_feature
   <222> (60)..(60)
   <223> n is a, c, g, or t
<220>
   <221> misc_feature
   <222> (63)..(63)
   <223> n is a, c, g, or t
<220>
   <221> misc_feature
   <222> (66)..(66)
   <223> n is a, c, g, or t
<220>
   <221> misc_feature
   <222> (69)..(69)
   <223> n is a, c, g, or t
<220>
   <221> misc_feature
   <222> (72)..(72)
   <223> n is a, c, g, or t
<220>
   <221> misc_feature
   <222> (75)..(75)
   <223> n is a, c, g, or t
<220>
   <221> misc_feature
   <222> (81)..(81)
   <223> n is a, c, g, or t
<220>
   <221> misc_feature
   <222> (84)..(84)
   <223> n is a, c, g, or t
<220>
   <221> misc_feature
   <222> (87)..(87)
   <223> n is a, c, g, or t
<220>
   <221> misc_feature
   <222> (90)..(90)
   <223> n is a, c, g, or t
<220>
   <221> misc_feature
   <222> (93)..(93)
   <223> n is a, c, g, or t
<220>
   <221> misc_feature
   <222> (99)..(99)
   <223> n is a, c, g, or t
<220>
   <221> misc_feature
   <222> (102)..(102)
   <223> n is a, c, g, or t
<220>
   <221> misc_feature
   <222> (105)..(105)
   <223> n is a, c, g, or t
<220>
   <221> misc_feature
   <222> (111)..(111)
   <223> n is a, c, g, or t
<220>
   <221> misc_feature
   <222> (114)..(114)
   <223> n is a, c, g, or t
<220>
   <221> misc_feature
   <222> (117)..(117)
   <223> n is a, c, g, or t
<220>
   <221> misc_feature
   <222> (120)..(120)
   <223> n is a, c, g, or t
<220>
   <221> misc_feature
   <222> (126)..(126)
   <223> n is a, c, g, or t
<220>
   <221> misc_feature
   <222> (132)..(132)
   <223> n is a, c, g, or t
<220>
   <221> misc_feature
   <222> (138)..(138)
   <223> n is a, c, g, or t
<220>
   <221> misc_feature
   <222> (141)..(141)
   <223> n is a, c, g, or t
<220>
   <221> misc_feature
   <222> (144)..(144)
   <223> n is a, c, g, or t
<220>
   <221> misc_feature
   <222> (150)..(150)
   <223> n is a, c, g, or t
<220>
   <221> misc_feature
   <222> (153)..(153)
   <223> n is a, c, g, or t
<220>
   <221> misc_feature
   <222> (156)..(156)
   <223> n is a, c, g, or t
<220>
   <221> misc_feature
   <222> (165)..(165)
   <223> n is a, c, g, or t
<220>
   <221> misc_feature
   <222> (180)..(180)
   <223> n is a, c, g, or t
<220>
   <221> misc_feature
   <222> (183)..(183)
   <223> n is a, c, g, or t
<220>
   <221> misc_feature
   <222> (186)..(186)
   <223> n is a, c, g, or t
<220>
   <221> misc_feature
   <222> (189)..(189)
   <223> n is a, c, g, or t
<220>
   <221> misc_feature
   <222> (192)..(192)
   <223> n is a, c, g, or t
<220>
   <221> misc_feature
   <222> (198)..(198)
   <223> n is a, c, g, or t
<220>
   <221> misc_feature
   <222> (201)..(201)
   <223> n is a, c, g, or t
<220>
   <221> misc_feature
   <222> (207)..(207)
   <223> n is a, c, g, or t
<220>
   <221> misc_feature
   <222> (213)..(213)
   <223> n is a, c, g, or t
<220>
   <221> misc_feature
   <222> (216)..(216)
   <223> n is a, c, g, or t
<220>
   <221> misc_feature
   <222> (219)..(219)
   <223> n is a, c, g, or t
<220>
   <221> misc_feature
   <222> (225)..(225)
   <223> n is a, c, g, or t
<220>
   <221> misc_feature
   <222> (228)..(228)
   <223> n is a, c, g, or t
<220>
   <221> misc_feature
   <222> (252)..(252)
   <223> n is a, c, g, or t
<220>
   <221> misc_feature
   <222> (276)..(276)
   <223> n is a, c, g, or t
<220>
   <221> misc_feature
   <222> (279)..(279)
   <223> n is a, c, g, or t
<220>
   <221> misc_feature
   <222> (282)..(282)
   <223> n is a, c, g, or t
<220>
   <221> misc_feature
   <222> (285)..(285)
   <223> n is a, c, g, or t
<220>
   <221> misc_feature
   <222> (294)..(294)
   <223> n is a, c, g, or t
<220>
   <221> misc_feature
   <222> (300)..(300)
   <223> n is a, c, g, or t
<220>
   <221> misc_feature
   <222> (315)..(315)
   <223> n is a, c, g, or t
<220>
   <221> misc_feature
   <222> (321)..(321)
   <223> n is a, c, g, or t
<220>
   <221> misc_feature
   <222> (327)..(327)
   <223> n is a, c, g, or t
<220>
   <221> misc_feature
   <222> (330)..(330)
   <223> n is a, c, g, or t
<220>
   <221> misc_feature
   <222> (339)..(339)
   <223> n is a, c, g, or t
<220>
   <221> misc_feature
   <222> (342)..(342)
   <223> n is a, c, g, or t
<220>
   <221> misc_feature
   <222> (345)..(345)
   <223> n is a, c, g, or t
<220>
   <221> misc_feature
   <222> (351)..(351)
   <223> n is a, c, g, or t
<220>
   <221> misc_feature
   <222> (354)..(354)
   <223> n is a, c, g, or t
<220>
   <221> misc_feature
   <222> (357)..(357)
   <223> n is a, c, g, or t
<220>
   <221> misc_feature
   <222> (363)..(363)
   <223> n is a, c, g, or t
<220>
   <221> misc_feature
   <222> (366)..(366)
   <223> n is a, c, g, or t
<220>
   <221> misc_feature
   <222> (378)..(378)
   <223> n is a, c, g, or t
<220>
   <221> misc_feature
   <222> (384)..(384)
   <223> n is a, c, g, or t
<220>
   <221> misc_feature
   <222> (399)..(399)
   <223> n is a, c, g, or t
<220>
   <221> misc_feature
   <222> (405)..(405)
   <223> n is a, c, g, or t
<220>
   <221> misc_feature
   <222> (411)..(411)
   <223> n is a, c, g, or t
<220>
   <221> misc_feature
   <222> (414)..(414)
   <223> n is a, c, g, or t
<220>
   <221> misc_feature
   <222> (417)..(417)
   <223> n is a, c, g, or t
<220>
   <221> misc_feature
   <222> (420)..(420)
   <223> n is a, c, g, or t
<220>
   <221> misc_feature
   <222> (423)..(423)
   <223> n is a, c, g, or t
<220>
   <221> misc_feature
   <222> (438)..(438)
   <223> n is a, c, g, or t
<220>
   <221> misc_feature
   <222> (447)..(447)
   <223> n is a, c, g, or t
<220>
   <221> misc_feature
   <222> (456)..(456)
   <223> n is a, c, g, or t
<220>
   <221> misc_feature
   <222> (465)..(465)
   <223> n is a, c, g, or t
<220>
   <221> misc_feature
   <222> (468)..(468)
   <223> n is a, c, g, or t
<220>
   <221> misc_feature
   <222> (474)..(474)
   <223> n is a, c, g, or t
<220>
   <221> misc_feature
   <222> (477)..(477)
   <223> n is a, c, g, or t
<220>
   <221> misc_feature
   <222> (480)..(480)
   <223> n is a, c, g, or t
<220>
   <221> misc_feature
   <222> (483)..(483)
   <223> n is a, c, g, or t
<220>
   <221> misc_feature
   <222> (489)..(489)
   <223> n is a, c, g, or t
<220>
   <221> misc_feature
   <222> (498)..(498)
   <223> n is a, c, g, or t
<220>
   <221> misc_feature
   <222> (501)..(501)
   <223> n is a, c, g, or t
<220>
   <221> misc_feature
   <222> (504)..(504)
   <223> n is a, c, g, or t
<220>
   <221> misc_feature
   <222> (513)..(513)
   <223> n is a, c, g, or t
<220>
   <221> misc_feature
   <222> (516)..(516)
   <223> n is a, c, g, or t
<220>
   <221> misc_feature
   <222> (525)..(525)
   <223> n is a, c, g, or t
<220>
   <221> misc_feature
   <222> (528)..(528)
   <223> n is a, c, g, or t
<220>
   <221> misc_feature
   <222> (531)..(531)
   <223> n is a, c, g, or t
<220>
   <221> misc_feature
   <222> (537)..(537)
   <223> n is a, c, g, or t
<220>
   <221> misc_feature
   <222> (546)..(546)
   <223> n is a, c, g, or t
<220>
   <221> misc_feature
   <222> (549)..(549)
   <223> n is a, c, g, or t
<220>
   <221> misc_feature
   <222> (552)..(552)
   <223> n is a, c, g, or t
<220>
   <221> misc_feature
   <222> (555)..(555)
   <223> n is a, c, g, or t
<220>
   <221> misc_feature
   <222> (564)..(564)
   <223> n is a, c, g, or t
<220>
   <221> misc_feature
   <222> (567)..(567)
   <223> n is a, c, g, or t
<220>
   <221> misc_feature
   <222> (570)..(570)
   <223> n is a, c, g, or t
<220>
   <221> misc_feature
   <222> (573)..(573)
   <223> n is a, c, g, or t
<220>
   <221> misc_feature
   <222> (576)..(576)
   <223> n is a, c, g, or t
<220>
   <221> misc_feature
   <222> (582)..(582)
   <223> n is a, c, g, or t
<220>
   <221> misc_feature
   <222> (585)..(585)
   <223> n is a, c, g, or t
<220>
   <221> misc_feature
   <222> (594)..(594)
   <223> n is a, c, g, or t
<220>
   <221> misc_feature
   <222> (597)..(597)
   <223> n is a, c, g, or t
<220>
   <221> misc_feature
   <222> (603)..(603)
   <223> n is a, c, g, or t
<220>
   <221> misc_feature
   <222> (606)..(606)
   <223> n is a, c, g, or t
<220>
   <221> misc_feature
   <222> (612)..(612)
   <223> n is a, c, g, or t
<220>
   <221> misc_feature
   <222> (615)..(615)
   <223> n is a, c, g, or t
<220>
   <221> misc_feature
   <222> (618)..(618)
   <223> n is a, c, g, or t
<220>
   <221> misc_feature
   <222> (627)..(627)
   <223> n is a, c, g, or t
<220>
   <221> misc_feature
   <222> (636)..(636)
   <223> n is a, c, g, or t
<220>
   <221> misc_feature
   <222> (639)..(639)
   <223> n is a, c, g, or t
<220>
   <221> misc_feature
   <222> (642)..(642)
   <223> n is a, c, g, or t
<220>
   <221> misc_feature
   <222> (645)..(645)
   <223> n is a, c, g, or t
<220>
   <221> misc_feature
   <222> (648)..(648)
   <223> n is a, c, g, or t
<220>
   <221> misc_feature
   <222> (651)..(651)
   <223> n is a, c, g, or t
<220>
   <221> misc_feature
   <222> (654)..(654)
   <223> n is a, c, g, or t
<220>
   <221> misc_feature
   <222> (660)..(660)
   <223> n is a, c, g, or t
<220>
   <221> misc_feature
   <222> (663)..(663)
   <223> n is a, c, g, or t
<220>
   <221> misc_feature
   <222> (666)..(666)
   <223> n is a, c, g, or t
<220>
   <221> misc_feature
   <222> (669)..(669)
   <223> n is a, c, g, or t
<220>
   <221> misc_feature
   <222> (672)..(672)
   <223> n is a, c, g, or t
<220>
   <221> misc_feature
   <222> (678)..(678)
   <223> n is a, c, g, or t
<220>
   <221> misc_feature
   <222> (693)..(693)
   <223> n is a, c, g, or t
<220>
   <221> misc_feature
   <222> (696)..(696)
   <223> n is a, c, g, or t
<220>
   <221> misc_feature
   <222> (702)..(702)
   <223> n is a, c, g, or t
<220>
   <221> misc_feature
   <222> (708)..(708)
   <223> n is a, c, g, or t
<220>
   <221> misc_feature
   <222> (714)..(714)
   <223> n is a, c, g, or t
<220>
   <221> misc_feature
   <222> (726)..(726)
   <223> n is a, c, g, or t
<220>
   <221> misc_feature
   <222> (729)..(729)
   <223> n is a, c, g, or t
<220>
   <221> misc_feature
   <222> (732)..(732)
   <223> n is a, c, g, or t
<220>
   <221> misc_feature
   <222> (741)..(741)
   <223> n is a, c, g, or t
<220>
   <221> misc_feature
   <222> (744)..(744)
   <223> n is a, c, g, or t
<220>
   <221> misc_feature
   <222> (747)..(747)
   <223> n is a, c, g, or t
<220>
   <221> misc_feature
   <222> (759)..(759)
   <223> n is a, c, g, or t
<220>
   <221> misc_feature
   <222> (762)..(762)
   <223> n is a, c, g, or t
<220>
   <221> misc_feature
   <222> (765)..(765)
   <223> n is a, c, g, or t
<220>
   <221> misc_feature
   <222> (768)..(768)
   <223> n is a, c, g, or t
<220>
   <221> misc_feature
   <222> (777)..(777)
   <223> n is a, c, g, or t
<220>
   <221> misc_feature
   <222> (783)..(783)
   <223> n is a, c, g, or t
<220>
   <221> misc_feature
   <222> (786)..(786)
   <223> n is a, c, g, or t
<220>
   <221> misc_feature
   <222> (801)..(801)
   <223> n is a, c, g, or t
<220>
   <221> misc_feature
   <222> (813)..(813)
   <223> n is a, c, g, or t
<220>
   <221> misc_feature
   <222> (816)..(816)
   <223> n is a, c, g, or t
<220>
   <221> misc_feature
   <222> (825)..(825)
   <223> n is a, c, g, or t
<220>
   <221> misc_feature
   <222> (840)..(840)
   <223> n is a, c, g, or t
<220>
   <221> misc_feature
   <222> (843)..(843)
   <223> n is a, c, g, or t
<220>
   <221> misc_feature
   <222> (849)..(849)
   <223> n is a, c, g, or t
<220>
   <221> misc_feature
   <222> (852)..(852)
   <223> n is a, c, g, or t
<220>
   <221> misc_feature
   <222> (870)..(870)
   <223> n is a, c, g, or t
<220>
   <221> misc_feature
   <222> (876)..(876)
   <223> n is a, c, g, or t
<220>
   <221> misc_feature
   <222> (879)..(879)
   <223> n is a, c, g, or t
<220>
   <221> misc_feature
   <222> (885)..(885)
   <223> n is a, c, g, or t
<220>
   <221> misc_feature
   <222> (888)..(888)
   <223> n is a, c, g, or t
<220>
   <221> misc_feature
   <222> (894)..(894)
   <223> n is a, c, g, or t
<220>
   <221> misc_feature
   <222> (900)..(900)
   <223> n is a, c, g, or t
<220>
   <221> misc_feature
   <222> (903)..(903)
   <223> n is a, c, g, or t
<220>
   <221> misc_feature
   <222> (906)..(906)
   <223> n is a, c, g, or t
<220>
   <221> misc_feature
   <222> (909)..(909)
   <223> n is a, c, g, or t
<220>
   <221> misc_feature
   <222> (912)..(912)
   <223> n is a, c, g, or t
<220>
   <221> misc_feature
   <222> (918)..(918)
   <223> n is a, c, g, or t
<220>
   <221> misc_feature
   <222> (927)..(927)
   <223> n is a, c, g, or t
<220>
   <221> misc_feature
   <222> (930)..(930)
   <223> n is a, c, g, or t
<220>
   <221> misc_feature
   <222> (936)..(936)
   <223> n is a, c, g, or t
<220>
   <221> misc_feature
   <222> (942)..(942)
   <223> n is a, c, g, or t
<220>
   <221> misc_feature
   <222> (948)..(948)
   <223> n is a, c, g, or t
<220>
   <221> misc_feature
   <222> (951)..(951)
   <223> n is a, c, g, or t
<220>
   <221> misc_feature
   <222> (957)..(957)
   <223> n is a, c, g, or t
<220>
   <221> misc_feature
   <222> (960)..(960)
   <223> n is a, c, g, or t
<220>
   <221> misc_feature
   <222> (966)..(966)
   <223> n is a, c, g, or t
<220>
   <221> misc_feature
   <222> (969)..(969)
   <223> n is a, c, g, or t
<220>
   <221> misc_feature
   <222> (972)..(972)
   <223> n is a, c, g, or t
<220>
   <221> misc_feature
   <222> (978)..(978)
   <223> n is a, c, g, or t
<220>
   <221> misc_feature
   <222> (981)..(981)
   <223> n is a, c, g, or t
<220>
   <221> misc_feature
   <222> (990)..(990)
   <223> n is a, c, g, or t
<220>
   <221> misc_feature
   <222> (1023)..(1023)
   <223> n is a, c, g, or t
<220>
   <221> misc_feature
   <222> (1038)..(1038)
   <223> n is a, c, g, or t
<220>
   <221> misc_feature
   <222> (1044)..(1044)
   <223> n is a, c, g, or t
<220>
   <221> misc_feature
   <222> (1047)..(1047)
   <223> n is a, c, g, or t
<220>
   <221> misc_feature
   <222> (1053)..(1053)
   <223> n is a, c, g, or t
<220>
   <221> misc_feature
   <222> (1056)..(1056)
   <223> n is a, c, g, or t
<220>
   <221> misc_feature
   <222> (1059)..(1059)
   <223> n is a, c, g, or t
<220>
   <221> misc_feature
   <222> (1062)..(1062)
   <223> n is a, c, g, or t
<220>
   <221> misc_feature
   <222> (1077)..(1077)
   <223> n is a, c, g, or t
<220>
   <221> misc_feature
   <222> (1095)..(1095)
   <223> n is a, c, g, or t
<220>
   <221> misc_feature
   <222> (1098)..(1098)
   <223> n is a, c, g, or t
<220>
   <221> misc_feature
   <222> (1101)..(1101)
   <223> n is a, c, g, or t
<220>
   <221> misc_feature
   <222> (1104)..(1104)
   <223> n is a, c, g, or t
<220>
   <221> misc_feature
   <222> (1107)..(1107)
   <223> n is a, c, g, or t
<220>
   <221> misc_feature
   <222> (1113)..(1113)
   <223> n is a, c, g, or t
<220>
   <221> misc_feature
   <222> (1116)..(1116)
   <223> n is a, c, g, or t
<220>
   <221> misc_feature
   <222> (1122)..(1122)
   <223> n is a, c, g, or t
<220>
   <221> misc_feature
   <222> (1131)..(1131)
   <223> n is a, c, g, or t
<220>
   <221> misc_feature
   <222> (1134)..(1134)
   <223> n is a, c, g, or t
<220>
   <221> misc_feature
   <222> (1137)..(1137)
   <223> n is a, c, g, or t
<220>
   <221> misc_feature
   <222> (1146)..(1146)
   <223> n is a, c, g, or t
<220>
   <221> misc_feature
   <222> (1155)..(1155)
   <223> n is a, c, g, or t
<220>
   <221> misc_feature
   <222> (1158)..(1158)
   <223> n is a, c, g, or t
<220>
   <221> misc_feature
   <222> (1164)..(1164)
   <223> n is a, c, g, or t
<220>
   <221> misc_feature
   <222> (1167)..(1167)
   <223> n is a, c, g, or t
<220>
   <221> misc_feature
   <222> (1173)..(1173)
   <223> n is a, c, g, or t
<220>
   <221> misc_feature
   <222> (1176)..(1176)
   <223> n is a, c, g, or t
<220>
   <221> misc_feature
   <222> (1179)..(1179)
   <223> n is a, c, g, or t
<220>
   <221> misc_feature
   <222> (1182)..(1182)
   <223> n is a, c, g, or t
<220>
   <221> misc_feature
   <222> (1191)..(1191)
   <223> n is a, c, g, or t
<220>
   <221> misc_feature
   <222> (1194)..(1194)
   <223> n is a, c, g, or t
<220>
   <221> misc_feature
   <222> (1200)..(1200)
   <223> n is a, c, g, or t
<220>
   <221> misc_feature
   <222> (1206)..(1206)
   <223> n is a, c, g, or t
<220>
   <221> misc_feature
   <222> (1212)..(1212)
   <223> n is a, c, g, or t
<220>
   <221> misc_feature
   <222> (1215)..(1215)
   <223> n is a, c, g, or t
<220>
   <221> misc_feature
   <222> (1218)..(1218)
   <223> n is a, c, g, or t
<220>
   <221> misc_feature
   <222> (1221)..(1221)
   <223> n is a, c, g, or t
<220>
   <221> misc_feature
   <222> (1224)..(1224)
   <223> n is a, c, g, or t
<220>
   <221> misc_feature
   <222> (1230)..(1230)
   <223> n is a, c, g, or t
<220>
   <221> misc_feature
   <222> (1233)..(1233)
   <223> n is a, c, g, or t
<220>
   <221> misc_feature
   <222> (1239)..(1239)
   <223> n is a, c, g, or t
<220>
   <221> misc_feature
   <222> (1242)..(1242)
   <223> n is a, c, g, or t
<220>
   <221> misc_feature
   <222> (1245)..(1245)
   <223> n is a, c, g, or t
<220>
   <221> misc_feature
   <222> (1248)..(1248)
   <223> n is a, c, g, or t
<220>
   <221> misc_feature
   <222> (1251)..(1251)
   <223> n is a, c, g, or t
<220>
   <221> misc_feature
   <222> (1254)..(1254)
   <223> n is a, c, g, or t
<220>
   <221> misc_feature
   <222> (1257)..(1257)
   <223> n is a, c, g, or t
<220>
   <221> misc_feature
   <222> (1266)..(1266)
   <223> n is a, c, g, or t
<220>
   <221> misc_feature
   <222> (1269)..(1269)
   <223> n is a, c, g, or t
<220>
   <221> misc_feature
   <222> (1278)..(1278)
   <223> n is a, c, g, or t
<220>
   <221> misc_feature
   <222> (1281)..(1281)
   <223> n is a, c, g, or t
<220>
   <221> misc_feature
   <222> (1299)..(1299)
   <223> n is a, c, g, or t
<220>
   <221> misc_feature
   <222> (1302)..(1302)
   <223> n is a, c, g, or t
<220>
   <221> misc_feature
   <222> (1305)..(1305)
   <223> n is a, c, g, or t
<220>
   <221> misc_feature
   <222> (1311)..(1311)
   <223> n is a, c, g, or t
<400> 5
   atggcnccnw snmgngcnaa yacnwsngtn athgtngtng gnggnggngg nacnathggn 60
   wsnwsnacng cnytncayyt ngtnmgnwsn ggntayacnc cnwsnaaygt nacngtnytn 120
   gaygcntayc cnathccnws nwsncarwsn gcnggnaayg ayytnaayaa rathatgggn 180
   gtnwsnytnm gnaayccngt ngayytncar ytngcnytng argcnmgnca ratgtggaay 240
   gargaygary tnttyaaraa rttyttycay aayacnggnm gnytngaytg ygcncayggn 300
   garaargaya thgcngayyt naarwsnggn taycargcny tngtngaygc nggnytngay 360
   gcnacnaayg artggytnga ywsngargay garathytna armgnatgcc nytnytnwsn 420
   mgngaycara thaarggntg gaargcnath ttywsnaarg ayggnggntg gytngcngcn 480
   gcnaargcna thaaygcngt nggngartay ytnmgngayc arggngtnmg nttyggntty 540
   tayggngcng gnwsnttyaa rgcnccnytn ytngcngarg gngtntgyat hggngtngar 600
   acngtngayg gnacnmgnta ytaygcngay aargtngtny tngcngcngg ngcntggwsn 660
   ccnacnytng tngarytnca ygarcartgy gtnwsnaarg cntgggtnta yggncayath 720
   carytnacnc cngargargc ngcnmgntay aaraaywsnc cngtngtnta yaayggngay 780
   gtnggnttyt tyttygarcc naaygarcay ggngtnatha argtntgyga ygarttyccn 840
   ggnttyacnm gnttyaarat gcaycarccn ttyggngcna argcnccnaa rmgnathwsn 900
   gtnccnmgnw sncaygcnaa rcayccnacn gayacnathc cngaygcnws ngaygtnwsn 960
   athmgnmgng cnathgcnac nttyatgccn carttyaara ayaaraarat gttyaaycar 1020
   gcnatgtgyt ggtgyacnga yacngcngay gcngcnytny tnathtgyga rcayccngar 1080
   tggaaraayt tygtnytngc nacnggngay wsnggncayw snttyaaryt nytnccnaay 1140
   athggnaarc aygtngtnga rytnytngar ggnacnytng cngaygayyt ngcncaygcn 1200
   tggmgntggm gnccnggnws nggngaygcn ytnaarwsnm gnmgnwsngc nccngcnaar 1260
   gayytngcng ayatgccngg ntggaaycay gayaarccnm gngcnaayyt ntag 1314
<210> 6
   <211> 35
   <212> DNA
   <213> Artificial Sequence
<220>
   <223> Primer for the substitution of nucleotides encoding glycine at position 182 of SEQ ID NO: 1
<400> 6
   gtgtacgttt cggtttctat gacgcgggct ctttc 35
<210> 7
   <211> 35
   <212> DNA
   <213> Artificial sequence
<220>
   <223> Primer for the substitution of nucleotides encoding glycine at position 182 of SEQ ID NO: 1
<400> 7
   gaaagagccc gcgtcataga aaccgaaacg tacac 35
<210> 8
   <211> 29
   <212> DNA
   <213> Artificial sequence
<220>
   <223> Primer for the substitution of the nucleotide encoding asparagine at position 268 of SEQ ID NO: 1
<400> 8
   ttttcttcga gccggacgag cacggcgtg 29
<210> 9
   <211> 29
   <212> DNA
   <213> Artificial sequence
<220>
   <223> Primer for the substitution of the nucleotide encoding asparagine at position 268 of SEQ ID NO: 1
<400> 9
   cacgccgtgc tcgtccggct cgaagaaaa 29
<210> 10
   <211> 37
   <212> DNA
   <213> Artificial sequence
<220>
   <223> Primer for the substitution of the nucleotide encoding histidine at position 384 of SEQ ID NO: 1
<400> 10
   ctgccaaata ttggcaagta tgttgttgaa ctgctgg 37
<210> 11
   <211> 37
   <212> DNA
   <213> Artificial sequence
<220>
   <223> Primer for the substitution of the nucleotide encoding histidine at position 384 of SEQ ID NO: 1
<400> 11
   ccagcagttc aacaacatac ttgccaatat ttggcag 37